(19) **Europäisches Patentamt**
**European Patent Office**
**Office européen des brevets**

(11) **EP 2 645 089 B1**

(12) **EUROPEAN PATENT SPECIFICATION**

(45) Date of publication and mention of the grant of the patent:
**05.12.2018 Bulletin 2018/49**

(51) Int Cl.:
**G01N 25/18** *(2006.01)*   **G01N 33/22** *(2006.01)*

(21) Application number: **13158708.1**

(22) Date of filing: **12.03.2013**

(54) **Calorific-value measuring system and calorific-value measuring method**

Kalorienwertmesssystem und Kalorienwertmessverfahren

Système et procédé de mesure de valeur calorifique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2012 JP 2012072258**

(43) Date of publication of application:
**02.10.2013 Bulletin 2013/40**

(73) Proprietor: **Azbil Corporation**
**Chiyoda-ku**
**Tokyo 100-6419 (JP)**

(72) Inventor: **Ooishi, Yasuharu**
**Tokyo, 100-6419 (JP)**

(74) Representative: **Klunker IP**
**Patentanwälte PartG mbB**
**Destouchesstraße 68**
**80796 München (DE)**

(56) References cited:
**EP-A1- 1 947 450**   **EP-A1- 2 345 891**
**WO-A1-02/40992**   **JP-A- 2012 233 859**

- **SMOLA A J ET AL: "A Tutorial on Support Vector Regression", NEUROCOLT TECHNICAL REPORT (NC-TR-98-030),, 1 October 1998 (1998-10-01), pages 1-122, XP002638775,**

## Description

Background

The present invention relates to a gas inspection technology. Specifically, the present invention relates to a calorific-value measuring system and a calorific-value measuring method.

Conventionally, in a case of obtaining a calorific value of mixed gas, it is necessary to use an expensive gas chromatograph or the like and to analyze components of the mixed gas. Further, for example, Japanese Patent Application Laid-open No. 2004-514138 discloses a method described in below. That is, thermal conductivity of mixed gas is measured, and sound speed in the mixed gas is measured. As a result, component fractions of methane ($CH_4$), propane ($C_3H_8$), nitrogen ($N_2$), and carbon dioxide ($CO_2$) in the mixed gas are calculated, respectively. The calorific value of the mixed gas is thus obtained.

Summary

However, the method disclosed in Japanese Patent Application Laid-open No. 2004-514138 not only requires a sensor for measuring thermal conductivity, but also requires an expensive sound-speed sensor for measuring sound speed. In view of this, it is an object of the present invention to provide a calorific-value measuring system and a calorific-value measuring method capable of easily measuring a calorific value of gas.

JP 2012/233859 A discloses a calorific value measuring system. The system includes a gas flowing in a pipe, a flow rate controlling device, a temperature measuring element in the pipe, a heater element, in the pipe, producing a plurality of temperatures, a measuring portion measuring a value from a temperature measuring element that is dependent on the temperature of the gas, and a value from the heater element at each of a plurality of temperatures. The system further includes an equation storage device storing a calorific value equation using values from the temperature measuring element and values from the heater element at the plurality of temperatures as independent variables and uses the calorific value as the dependent variable, and a calorific value portion calculating a value for the calorific value of a gas through substituting a value from the temperature measuring element and a value from the heater element into the independent variables of the calorific value equation.

According to EP 1 947 450 A1, a heat radiation coefficient C from a microheater is calculated in accordance with a power applied to the microheater which is supported in air and provided in an ambient gas, a heater temperature, and an ambient temperature at this moment. Further, a thermal conductivity of the ambient gas is obtained from the calculated heat radiation coefficient based on a proportional relation between a thermal conductivity of the ambient gas and the heat radiation coefficient at a measurement temperature.

JP2012202739 discloses a related measurement system in a chamber connected to a pipe, and discloses the use of a pressure sensor signal for a calorific value equation calculation, additionally in a comparative example an equation is used by excluding the independent variable of an electric signal SP from a pressure sensor.

In the present invention, there is provided a calorific-value-equation creating system and method, according to independent claim 1 and 5.

In the present invention, a calorific-value measuring system and a calorific-value measuring method according to independent claims 9 and 13 are provided capable of easily measuring a calorific value of gas.

Brief Description of Drawings

Fig. 1 is a perspective view showing a first microchip according to an embodiment of the present invention;

Fig. 2 is a sectional view of the first microchip according to the embodiment of the present invention, which is taken along the line II-II of Fig. 1;

Fig. 3 is a perspective view showing a second microchip according to the embodiment of the present invention;

Fig. 4 is a sectional view of the second microchip according to the embodiment of the present invention, which is taken along the line IV-IV of Fig. 3;

Fig. 5 is a circuit diagram showing a heating element according to the embodiment of the present invention;

Fig. 6 is a circuit diagram showing a temperature detector according to the embodiment of the present invention;

Fig. 7 is a graph showing relation between thermal conductivity and heat-radiation coefficients according to the embodiment of the present invention;

Fig. 8 is a graph showing relation between heat-radiation coefficients of gas and temperature of the heating element according to the embodiment of the present invention;

Fig. 9 is a first graph showing relation between thermal conductivity and resistance of the heating element according to the embodiment of the present invention;

Fig. 10 is a second graph showing relation between thermal conductivity and resistance of the heating element according to the embodiment of the present invention;

Fig. 11 is a third graph showing relation between thermal conductivity and resistance of the heating element according to the embodiment of the present invention;

Fig. 12 is a fourth graph showing relation between thermal conductivity and resistance of the heating element according to the embodiment of the present invention;

Fig. 13 is a first graph showing relation between thermal conductivity and electric power for driving the heating element according to the embodiment of the present invention;

Fig. 14 is a second graph showing relation between thermal conductivity and electric power for driving the heating element according to the embodiment of the present invention;

Fig. 15 is a first schematic diagram showing a calorific-value measuring system according to the embodiment of the present invention;

Fig. 16 is a second schematic diagram showing the calorific-value measuring system according to the embodiment of the present invention;

Fig. 17 is a first schematic diagram showing how the microchip is arranged according to the embodiment of the present invention;

Fig. 18 is a second schematic diagram showing how the microchip is arranged according to the embodiment of the present invention;

Fig. 19 is a flowchart showing a calorific-value-equation creating method according to the embodiment of the present invention;

Fig. 20 is a flowchart showing a calorific-value measuring method according to the embodiment of the present invention;

Fig. 21 is a graph showing calculation errors of a calorific value according to Example 1 of the present invention;

Fig. 22 is a graph showing calculation errors of a calorific value according to a comparative example of the present invention;

Fig. 23 is a graph showing calculated calorific values according to Example 2 of the present invention;

Fig. 24 is a schematic diagram showing how the microchip is arranged according to Example 3 of the present invention;

Fig. 25 is a schematic diagram showing how the microchip is arranged according to a comparative example of the present invention;

Fig. 26 is a graph showing responsivity of a calorific-value measuring system according to Example 3 of the present invention;

Fig. 27 is a graph showing responsivity of a calorific-value measuring system according to a comparative example of the present invention; and

Fig. 28 is a graph showing relation between a flow rate of measuring-target mixed gas and errors of a calculated calorific value according to Example 3 of the present invention.

Detailed Description of Embodiments

Hereinafter, embodiments of the present invention will be described with reference to the drawings. In the drawings, the same or similar parts are denoted by the same or similar reference symbols. Note that the drawings are schematic drawings. Specific dimensions and the like are to be determined with reference to the following description. As a matter of course, dimensional relation between one figure and another figure may be different, and a dimensional ratio between one figure and another figure may be different.

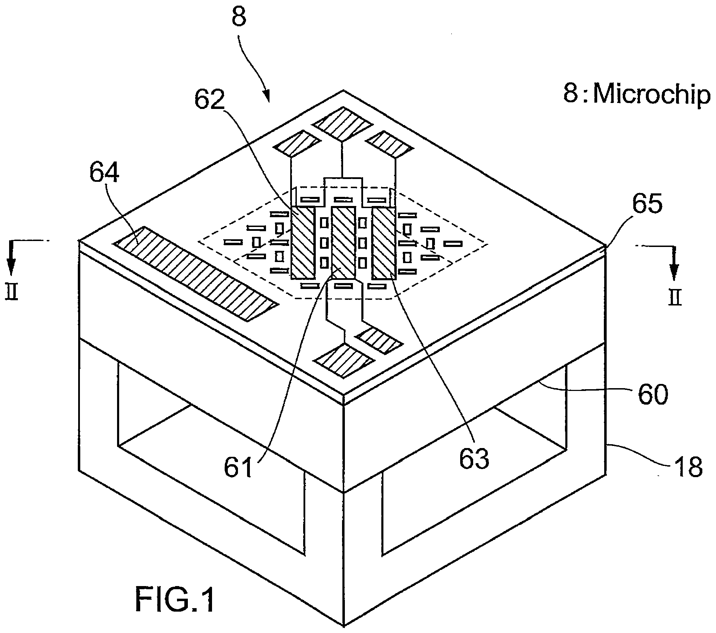

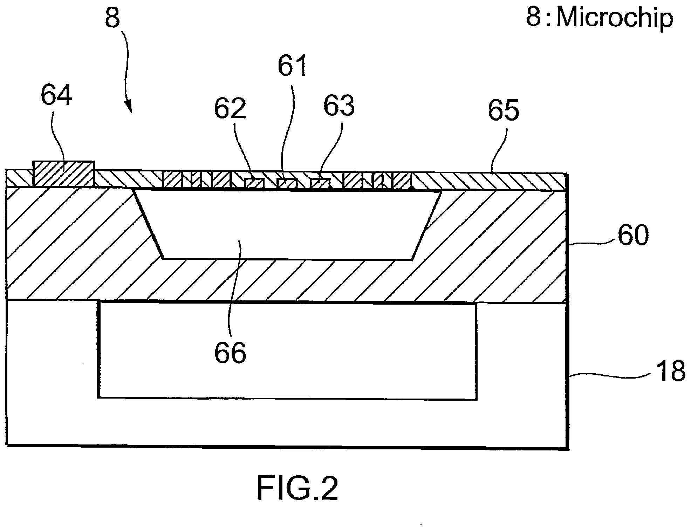

Fig. 1 is a perspective view showing a microchip 8. Fig. 2 is a sectional view of the microchip 8, which is taken along the line II-II. First, with reference to Fig. 1 and Fig. 2, the microchip 8 used in a calorific-value measuring system according to an embodiment will be described. The microchip 8 includes a substrate 60 and an insulating film 65. The substrate 60 has a cavity 66. The insulating film 65 is arranged on the substrate 60 such that the insulating film 65 covers the cavity 66. The thickness of the substrate 60 is, for example, 0.5 mm. Further, the length of the substrate 60 is, for example, about 1.5 mm. The width of the substrate 60 is, for example, about 1.5 mm. Part of the insulating film 65, which covers the cavity 66, is a thermal-insulating diaphragm. The microchip 8 further includes a heating element 61, a first temperature detector 62, a second temperature detector 63, and a heat-retention device 64. The heating element 61, the first temperature detector 62, and the second temperature detector 63 are provided in the diaphragm portion of the insulating film 65. The heating element 61 is arranged between the first temperature detector 62 and the second temperature detector 63. The heat-retention device 64 is provided on the substrate 60.

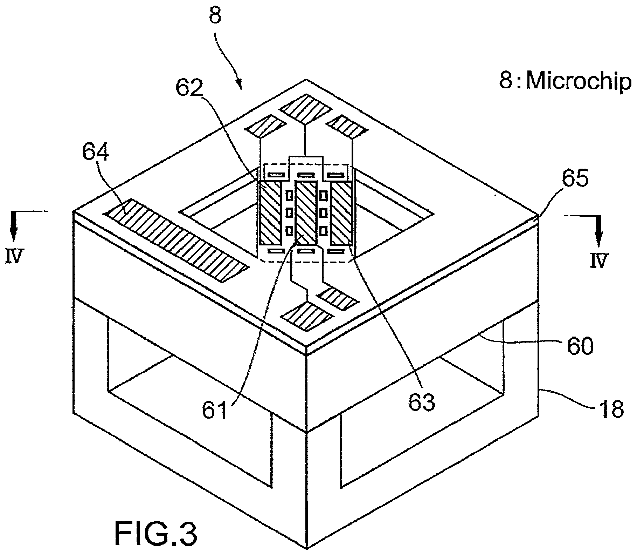

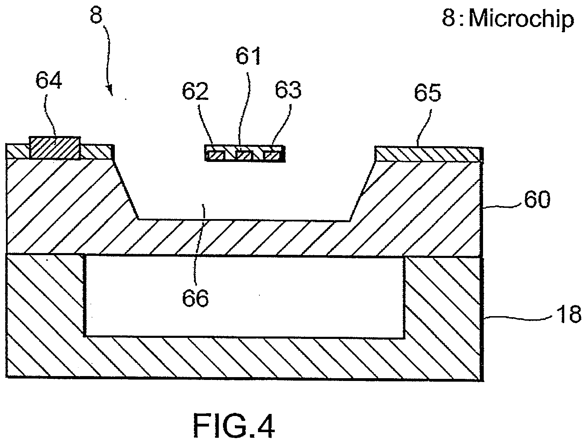

The diaphragm has a plurality of holes. Since the diaphragm has the plurality of holes, gas in the cavity 66 is exchanged rapidly. Alternatively, as shown in Fig. 3 and Fig. 4 (sectional view taken along the line IV-IV of Fig. 3), the insulating film 65 may be arranged on the substrate 60 such that the insulating film 65 covers the cavity 66 in a bridge-like manner. Part of the cavity 66 is thus exposed, and gas in the cavity 66 is exchanged rapidly.

The heating element 61 is arranged at the center of the diaphragm portion of the insulating film 65, which covers the cavity 66. The heating element 61 is, for example, a resistor. The heating element 61 receives electric power, produces heat, and heats the atmosphere gas exposed to the heating element 61. Each of the first temperature detector 62 and the second temperature detector 63 is, for example, an electronic device such as a passive device. An example of the passive device is a resistor. Each of the first temperature detector 62 and the second temperature detector 63 outputs an electric signal depending on the gas temperature of the atmosphere gas. Hereinafter, an example, not forming an embodiment of the invention, in which a signal output from the first temperature detector 62 is used, will be described. However, the embodiment is not limited to this. For example, an average value of a signal output from the first temperature detector 62 and a signal output from the second temperature detector 63 may be used as a signal output from the temperature detectors.

The heat-retention device 64 is, for example, a resistor. The heat-retention device 64 receives electric power, produces heat, and keeps the temperature of the substrate 60 constant. The substrate 60 may be made from silicon (Si) or the like. The insulating film 65 may be made from silicon oxide ($SiO_2$) or the like. The cavity 66 is formed by means of anisotropic etching or the like. Further, each of the heating element 61, the first temperature detector 62, the second temperature detector 63, and the heat-retention device 64 may be made from platinum (Pt) or the like, and may be formed by means of lithography or the like. Further, the heating element 61, the first temperature detector 62, and the second temperature detector 63 may be the same components.

A heat-insulating member 18 is arranged on a bottom surface of the microchip 8. The microchip 8 is fixed on a container such as a chamber, which is filled with atmosphere gas, via the heat-insulating member 18. Since the microchip 8 is fixed on the container via the heat-insulating member 18, the temperature of the microchip 8 is unlikely to be affected by temperature fluctuation of an inner wall of the container. The heat-insulating member 18 is made from glass or the like. The thermal conductivity of the heat-insulating member 18 is, for example, 1.0 W/(m·K) or less.

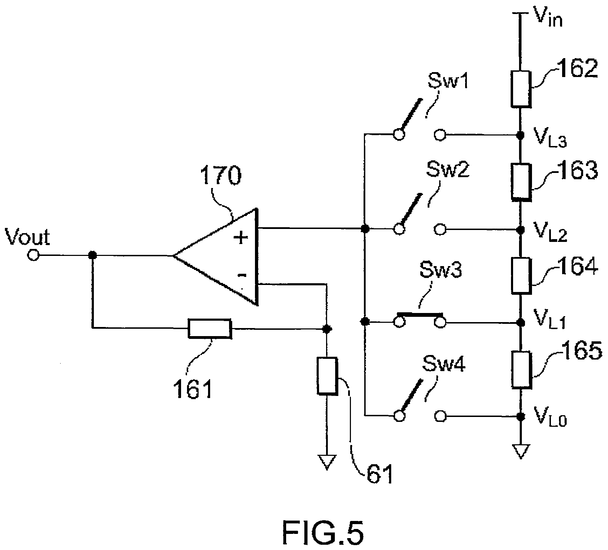

As shown in Fig. 5, for example, a - input terminal of an operational amplifier 170 is electrically connected to one end of the heating element 61. The other end of the heating element 61 is grounded. Further, a resistor 161 is connected in parallel with the - input terminal and an output terminal of the operational amplifier 170. A + input terminal of the operational amplifier 170 is electrically connected between a resistor 162 and a resistor 163, between the resistor 163 and a resistor 164, between the resistor 164 and a resistor 165, or to a grounded terminal of the resistor 165. The resistor 162, the resistor 163, the resistor 164, and the resistor 165 are connected in series. Each of the resistors 162 to 165 has a predetermined resistance value. A voltage $V_{in}$ is applied to one end of the resistor 162, for example. In this case, according to this structure, a first voltage $V_{L1}$ is generated between the resistor 165 and the resistor 164. A second voltage $V_{L2}$ is generated between the resistor 164 and the resistor 163. The second voltage $V_{L2}$ is higher than the first voltage $V_{L1}$. A third voltage $V_{L3}$ is generated between the resistor 163 and the resistor 162. The third voltage $V_{L3}$ is higher than the second voltage $V_{L2}$.

A switch Sw1 is provided between a line connecting the resistor 162 and the resistor 163, and the + input terminal of the operational amplifier. A switch Sw2 is provided between a line connecting the resistor 163 and the resistor 164, and the + input terminal of the operational amplifier. A switch Sw3 is provided between a line connecting the resistor 164 and the resistor 165, and the + input terminal of the operational amplifier. A switch Sw4 is provided between the grounded terminal of the resistor 165 and the + input terminal of the operational amplifier.

In a case where the third voltage $V_{L3}$ is applied to the + input terminal of the operational amplifier 170, a current passes only through the switch Sw1, and the switches Sw2, Sw3, and Sw4 are not connected. In a case where the second voltage $V_{L2}$ is applied to the + input terminal of the operational amplifier 170, a current passes only through the switch Sw2, and the switches Sw1, Sw3, and Sw4 are not connected. In a case where the first voltage $V_{L1}$ is applied to the + input terminal of the operational amplifier 170, a current passes only through the switch Sw3, and the switches Sw1, Sw2, and Sw4 are not connected. In a case where a voltage $V_{L0}$ (0 V) is applied to the + input terminal of the operational amplifier 170, a current passes only through the switch Sw4, and the switches Sw1, Sw2, and Sw3 are not connected. In this manner, it is possible to apply 0 V or one of the three levels of voltages to the + input terminal of the operational amplifier 170, by opening/closing the switches Sw1, Sw2, Sw3, and Sw4. That is, it is possible to set applied voltage, which determines a heating temperature of the heating element 61, at three levels, by opening/closing the switches Sw1, Sw2, Sw3, and Sw4.

Here, $T_{H1}$ is indicative of the temperature of the heating element 61 in a case where the first voltage $V_{L1}$ is applied to the + input terminal of the operational amplifier 170. $T_{H2}$ is indicative of the temperature of the heating element 61 in a case where the second voltage $V_{L2}$ is applied to the + input terminal of the operational amplifier 170. $T_{H3}$ is indicative of the temperature of the heating element 61 in a case where the third voltage $V_{L3}$ is applied to the + input terminal of the operational amplifier 170.

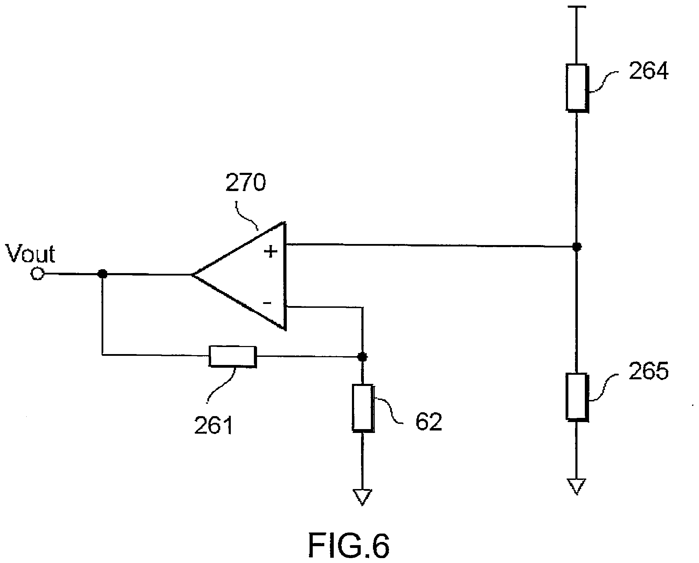

As shown in Fig. 6, for example, a - input terminal of an operational amplifier 270 is electrically connected to one end of the first temperature detector 62. The other end of the first temperature detector 62 is grounded. Further, a resistor 261 is connected in parallel with the - input terminal and an output terminal of the operational amplifier 270. A + input terminal of the operational amplifier 270 is electrically connected to a line connecting a resistor 264 and a resistor 265. The resistor 264 and the resistor 265 are connected in series. According to this structure, a small voltage (about 0.3 V) is applied to the first temperature detector 62.

The resistance value of the heating element 61 of Fig. 1 and Fig. 2 depends on the temperature of the heating element 61. The following Equation (1) shows the relation between the temperature $T_H$ of the heating element 61 and the resistance value $R_H$ of the heating element 61.

$$R_H = R_{H\_STD} \times [1 + \alpha_H (T_H - T_{H\_STD}) + \beta_H (T_H - T_{H\_STD})^2] \quad \ldots (1)$$

Here, $T_{H\_STD}$ is indicative of a standard temperature of the heating element 61, and is, for example, 20°C. $R_{H\_STD}$ is indicative of a previously-measured resistance value of the heating element 61 at the standard temperature $T_{H\_STD}$. $\alpha_H$ is indicative of a primary resistance temperature coefficient. $\beta_H$ is indicative of a secondary resistance temperature coefficient.

As shown in the following Equation (2), the resistance value $R_H$ of the heating element 61 is obtained based on the electric power $P_H$ for driving the heating element 61 and based on the current $I_H$ passing through the heating element 61.

$$R_H = P_H / I_H^2 \quad \ldots (2)$$

Alternatively, as shown in the following Equation (3), the resistance value $R_H$ of the heating element 61 is obtained based on the voltage $V_H$ applied to the heating element 61 and based on the current $I_H$ passing through the heating element 61.

$$R_H = V_H / I_H \quad \ldots (3)$$

Here, the temperature $T_H$ of the heating element 61 is stable when the heating element 61 and the atmosphere gas are thermally balanced. Note that the thermally balanced status means a status in which heat produced by the heating element 61 and heat radiated from the heating element 61 to the atmosphere gas are balanced. As shown in the Equation (4), the electric power $P_H$ for driving the heating element 61 under the balanced status is divided by $\Delta T_H$. $\Delta T_H$ is a difference between the temperature $T_H$ of the heating element 61 and the temperature $T_I$ of the atmosphere gas. As a result, the heat-radiation coefficient $M_I$ (e.g., W/°C) of the atmosphere gas is obtained.

$$M_I = P_H / (T_H - T_I)$$
$$= P_H / \Delta T_H = (V_H^2 / R_H) / \Delta T_H \quad \ldots (4)$$

Based on Equation (1), the following Equation (5) shows the temperature $T_H$ of the heating element 61.

$$T_H = (1/2\beta_H) \times [-\alpha_H + [\alpha_H^2 - 4\beta_H (1 - R_H / R_{H\_STD})]^{1/2}] + T_{H\_STD} \quad \ldots (5)$$

In view of this, the following Equation (6) shows the difference $\Delta T_H$ between the temperature $T_H$ of the heating element 61 and the temperature $T_I$ of the atmosphere gas.

$$\Delta T_H = (1/2\beta_H) \times [-\alpha_H + [\alpha_H^2 - 4\beta_H (1 - R_H / R_{H\_STD})]^{1/2}] + T_{H\_STD} - T_I \quad \ldots (6)$$

The temperature $T_I$ of the atmosphere gas is approximate to the temperature $T_I$ of the first temperature detector 62. Electric power is supplied to the first temperature detector 62 such that the first temperature detector 62 does not produce heat by itself. The following Equation (7) shows the relation between the temperature $T_I$ of the first temperature detector 62 and the resistance value $R_I$ of the first temperature detector 62.

$$R_I = R_{I\_STD} \times [1 + \alpha_I (T_I - T_{I\_STD}) + \beta_I (T_I - T_{I\_STD})^2] \quad \ldots (7)$$

$T_{I\_STD}$ is indicative of a standard temperature of the first temperature detector 62 and is, for example, 20°C. $R_{I\_STD}$ is indicative of a previously-measured resistance value of the first temperature detector 62 at the standard temperature $T_{I\_STD}$. $\alpha_I$ is indicative of a primary resistance temperature coefficient. $\beta_I$ is indicative of a secondary resistance temperature coefficient. In view of Equation (7), the following Equation (8) shows the temperature $T_I$ of the first temperature detector 62.

$$T_I=(1/2\beta_I)\times[-\alpha_I+[\alpha_I{}^2-4\beta_I(1-R_I/R_{I\_STD})]^{1/2}]+T_{I\_STD} \ \ldots(8)$$

Accordingly, the following Equation (9) shows the heat-radiation coefficient $M_I$ of the atmosphere gas.

$$M_I=P_H/\Delta T_H$$
$$=P_H/[(1/2\beta_H)[-\alpha_H+[\alpha_H{}^2-4\beta_H(1-R_H/R_{H\_STD})]^{1/2}]+T_{H\_STD}-(1/2\beta_I)[-\alpha_I+[\alpha_I{}^2-4\beta_I(1-R_I/R_{I\_STD})]^{1/2}]-T_{I\_STD}] \ \ldots(9)$$

It is possible to measure the current $I_H$ passing through the heating element 61 and the electric power $P_H$ for driving the heating element 61 or the voltage $V_H$ applied to the heating element 61. Therefore it is possible to calculate the resistance value $R_H$ of the heating element 61 by using Equation (2) or Equation (3). Similarly, it is possible to calculate the resistance value $R_I$ of the first temperature detector 62. Therefore the microchip 8 is capable of calculating the heat-radiation coefficient $M_I$ of the atmosphere gas by using Equation (9).

Note that the heat-retention device 64 keeps the temperature of the substrate 60 constant. As a result, before the heating element 61 produces heat, the temperature of the atmosphere gas around the microchip 8 is approximate to the constant temperature of the substrate 60. Because of this, fluctuation of the temperature of the atmosphere gas is reduced before the heating element 61 produces heat. The heating element 61 further heats the atmosphere gas, whose temperature fluctuation is once reduced. As a result, it is possible to calculate the heat-radiation coefficient $M_I$ more accurately.

Here, the atmosphere gas is mixed gas including four gas components, i.e., gas A, gas B, gas C, and gas D. As shown in the following Equation (10), the sum of the volume fraction $V_A$ of the gas A, the volume fraction $V_B$ of the gas B, the volume fraction $V_C$ of the gas C, and the volume fraction $V_D$ of the gas D is 1.

$$V_A+V_B+V_C+V_D=1 \ \ldots(10)$$

Further, $K_A$ is indicative of the calorific value of the gas A per unit volume. $K_B$ is indicative of the calorific value of the gas B per unit volume. $K_C$ is indicative of the calorific value of the gas C per unit volume. $K_D$ is indicative of the calorific value of the gas D per unit volume. Q is indicative of the calorific value of the mixed gas per unit volume. In this case, Q equals to the sum of values, which are obtained by multiplying the volume fractions of the gas components by the calorific values of heat produced by the gas components per unit volume, respectively. That is, the following Equation (11) shows the calorific value Q (e.g., MJ/m³) of the mixed gas per unit volume.

$$Q=K_A\times V_A+K_B\times V_B+K_C\times V_C+K_D\times V_D \ \ldots(11)$$

Further, $C_A$ is indicative of the thermal conductivity of the gas A per unit volume. $C_B$ is indicative of the thermal conductivity of the gas B per unit volume. $C_C$ is indicative of the thermal conductivity of the gas C per unit volume. $C_D$ is indicative of the thermal conductivity of the gas D per unit volume. $C_I$ is indicative of the thermal conductivity of the mixed gas per unit volume. In this case, $C_I$ equals to the sum of values, which are obtained by multiplying the volume fractions of the gas components by the thermal conductivities of the gas components per unit volume, respectively. That is, the following Equation (12) shows the thermal conductivity $C_I$ (e.g., W/(mK)) of the mixed gas per unit volume.

$$C_I=C_A\times V_A+C_B\times V_B+C_C\times V_C+C_D\times V_D \ \ldots(12)$$

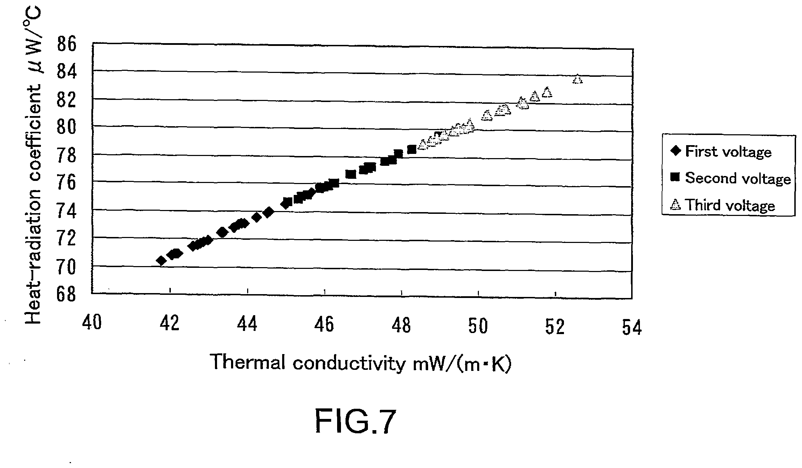

Fig. 7 is a graph showing the relation between the thermal conductivity and the heat-radiation coefficient. Fig. 7 shows that the first voltage $V_1$, the second voltage $V_2$, and the third voltage $V_3$ are applied to the heating element 61. The second voltage $V_2$ is higher than the first voltage $V_1$. The third voltage $V_3$ is higher than the second voltage $V_2$. As

shown in Fig. 7, the thermal conductivity is in proportion to the heat-radiation coefficient, in general. $M_A$ is indicative of the heat-radiation coefficient of the gas A. $M_B$ is indicative of the heat-radiation coefficient of the gas B. $M_C$ is indicative of the heat-radiation coefficient of the gas C. $M_D$ is indicative of the heat-radiation coefficient of the gas D. $M_I$ is indicative of the heat-radiation coefficient of the mixed gas. In this case, $M_I$ equals to the sum of values, which are obtained by multiplying the volume fractions of the gas components by the heat-radiation coefficients of the gas components, respectively. That is, the following Equation (13) shows the heat-radiation coefficient $M_I$ of the mixed gas.

$$M_I = M_A \times V_A + M_B \times V_B + M_C \times V_C + M_D \times V_D \quad \ldots (13)$$

Further, the heat-radiation coefficient of gas depends on the temperature $T_H$ of the heating element 61. So the following Equation (14) shows the heat-radiation coefficient $M_I$ of the mixed gas, as a function of the temperature $T_H$ of the heating element 61.

$$M_I(T_H) = M_A(T_H) \times V_A + M_B(T_H) \times V_B + M_C(T_H) \times V_C + M_D(T_H) \times V_D \quad \ldots (14)$$

That is, the following Equation (15) shows the heat-radiation coefficient $M_{I1}$ of the mixed gas ($T_{H1}$) where $T_{H1}$ is indicative of the temperature of the heating element 61. The following Equation (16) shows the heat-radiation coefficient $M_{I2}$ of the mixed gas ($T_{H2}$) where $T_{H2}$ is indicative of the temperature of the heating element 61. The following Equation (17) shows the heat-radiation coefficient $M_{I3}$ of the mixed gas ($T_{H3}$) where $T_{H3}$ is indicative of the temperature of the heating element 61.

$$M_{I1}(T_{H1}) = M_A(T_{H1}) \times V_A + M_B(T_{H1}) \times V_B + M_C(T_{H1}) \times V_C + M_D(T_{H1}) \times V_D \quad \ldots (15)$$

$$M_{I2}(T_{H2}) = M_A(T_{H2}) \times V_A + M_B(T_{H2}) \times V_B + M_C(T_{H2}) \times V_C + M_D(T_{H2}) \times V_D \quad \ldots (16)$$

$$M_{I3}(T_{H3}) = M_A(T_{H3}) \times V_A + M_B(T_{H3}) \times V_B + M_C(T_{H3}) \times V_C + M_D(T_{H3}) \times V_D \quad \ldots (17)$$

Here, the heat-radiation coefficients $M_A(T_H)$, $M_B(T_H)$, $M_C(T_H)$, $M_D(T_H)$ of the respective gas components are non-linear with respect to the temperature $T_H$ of the heating element 61. In this case, each of Equations (15) to (17) has a linear-independent relation. Further, the heat-radiation coefficients $M_A(T_H)$, $M_B(T_H)$, $M_C(T_H)$, $M_D(T_H)$ of the respective gas components are linear with respect to the temperature $T_H$ of the heating element 61. In addition, change rates of the heat-radiation coefficients $M_A(T_H)$, $M_B(T_H)$, $M_C(T_H)$, $M_D(T_H)$ of the respective gas components with respect to the temperature $T_H$ of the heating element 61 are different from each other. Also in this case, each of Equations (15) to (17) has a linear-independent relation. Further, in a case where each of Equations (15) to (17) has a linear-independent relation, each of Equation (10) and Equations (15) to (17) has a linear-independent relation.

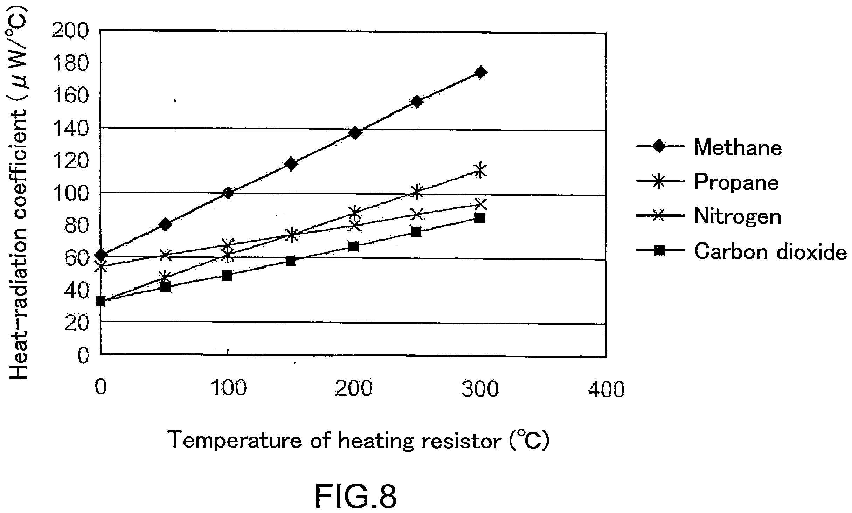

Fig. 8 is a graph showing relation between heat-radiation coefficients of methane ($CH_4$), propane ($C_3H_8$), nitrogen ($N_2$), and carbon dioxide ($CO_2$) and the temperature of the heating element 61. Methane ($CH_4$), propane ($C_3H_8$), nitrogen ($N_2$), and carbon dioxide ($CO_2$) are included in natural gas. The heating element 61 is a heating resistor. The heat-radiation coefficient of each of the gas components (methane ($CH_4$), propane ($C_3H_8$), nitrogen ($N_2$), and carbon dioxide ($CO_2$)) is linear with respect to the temperature of the heating element 61. However, the change rates of the heat-radiation coefficients of methane ($CH_4$), propane ($C_3H_8$), nitrogen ($N_2$), and carbon dioxide ($CO_2$) with respect to the temperature of the heating element 61 are different from each other. In view of this, in the case where the mixed gas includes methane ($CH_4$), propane ($C_3H_8$), nitrogen ($N_2$), and carbon dioxide ($CO_2$) as gas components, each of Equations (15) to (17) has a linear-independent relation.

The heat-radiation coefficients $M_A(T_{H1})$, $M_B(T_{H1})$, $M_C(T_{H1})$, $M_D(T_{H1})$, $M_A(T_{H2})$, $M_B(T_{H2})$, $M_C(T_{H2})$, $M_D(T_{H2})$, $M_A(T_{H3})$, $M_B(T_{H3})$, $M_C(T_{H3})$, $M_D(T_{H3})$ of the gas components of Equations (15) to (17) may be previously obtained by measuring or the like. In view of this, by solving a simultaneous equation including Equation (10) and Equations (15) to (17), the volume fraction $V_A$ of the gas A, the volume fraction $V_B$ of the gas B, the volume fraction $V_C$ of the gas C, and the volume fraction $V_D$ of the gas D are obtained. As shown in each of the following Equations (18) to (21), each of the volume fraction $V_A$ of the gas A, the volume fraction $V_B$ of the gas B, the volume fraction $V_C$ of the gas C, and the volume fraction $V_D$ of the gas D is obtained as a function of the heat-radiation coefficients $M_{I1}(T_{H1})$, $M_{I2}(T_{H2})$, $M_{I3}(T_{H3})$ of the mixed gas. Note that, in the following Equations (18) to (21), n is indicative of a natural number, and $f_n$ is indicative of a function.

$$V_A = f_1[M_{I1}(T_{H1}), \ M_{I2}(T_{H2}), \ M_{I3}(T_{H3})] \ \ldots (18)$$

$$V_B = f_2[M_{I1}(T_{H1}), \ M_{I2}(T_{H2}), \ M_{I3}(T_{H3})] \ \ldots (19)$$

$$V_C = f_3[M_{I1}(T_{H1}), \ M_{I2}(T_{H2}), \ M_{I3}(T_{H3})] \ \ldots (20)$$

$$V_D = f_4[M_{I1}(T_{H1}), \ M_{I2}(T_{H2}), \ M_{I3}(T_{H3})] \ \ldots (21)$$

Here, Equations (18) to (21) are substituted in Equation (11). As a result, the following Equation (22) is obtained.

$$
\begin{aligned}
Q &= K_A \times V_A + K_B \times V_B + K_C \times V_C + K_D \times V_D \\
&= K_A \times f_1[M_{I1}(T_{H1}), \ M_{I2}(T_{H2}), \ M_{I3}(T_{H3})] \\
&+ K_B \times f_2[M_{I1}(T_{H1}), \ M_{I2}(T_{H2}), \ M_{I3}(T_{H3})] \\
&+ K_C \times f_3[M_{I1}(T_{H1}), \ M_{I2}(T_{H2}), \ M_{I3}(T_{H3})] \\
&+ K_D \times f_4[M_{I1}(T_{H1}), \ M_{I2}(T_{H2}), \ M_{I3}(T_{H3})] \ \ldots (22)
\end{aligned}
$$

As shown in Equation (22), the calorific value Q of the mixed gas per unit volume is obtained based on an equation in which the heat-radiation coefficients $M_{I1}(T_{H1})$, $M_{I2}(T_{H2})$, $M_{I3}(T_{H3})$ of the mixed gas are variables. The heat-radiation coefficients $M_{I1}(T_{H1})$, $M_{I2}(T_{H2})$, $M_{I3}(T_{H3})$ of the mixed gas are values in the case where the temperatures of the heating element 61 are $T_{H1}$, $T_{H2}$, and $T_{H3}$, respectively. In view of this, the following Equation (23) shows the calorific value Q of the mixed gas. $g_1$ is indicative of a function.

$$Q = g_1[M_{I1}(T_{H1}), \ M_{I2}(T_{H2}), \ M_{I3}(T_{H3})] \ \ldots (23)$$

In view of this, the inventors have found out the following fact. That is, it is possible to easily calculate the calorific value Q produced by measuring-target mixed gas, per unit volume, by previously obtaining Equation (23) about the mixed gas including the gas A, the gas B, the gas C, and the gas D, even if the volume fraction $V_A$ of the gas A, the volume fraction $V_B$ of the gas B, the volume fraction $V_C$ of the gas C, and the volume fraction $V_D$ of the gas D are unknown. Specifically, the heat-radiation coefficients $M_{I1}(T_{H1})$, $M_{I2}(T_{H2})$, $M_{I3}(T_{H3})$ of the measuring-target mixed gas in the case where the heating temperatures of the heating element 61 are $T_{H1}$, $T_{H2}$, and $T_{H3}$, respectively, are measured by using Equation (9). The measured $M_{I1}(T_{H1})$, $M_{I2}(T_{H2})$, $M_{I3}(T_{H3})$ are substituted in Equation (23). As a result, it is possible to uniquely obtain the calorific value Q produced by the measuring-target mixed gas.

According to the above-mentioned method, not forming an embodiment of the invention, the heat-radiation coefficients $M_{I1}(T_{H1})$, $M_{I2}(T_{H2})$, $M_{I3}(T_{H3})$ of the measuring-target mixed gas are measured by using the heating element 61 and the first temperature detector 62 of the microchip 8. As a result, the calorific value Q is obtained. Meanwhile, according to the following method, it is possible to obtain the calorific value Q produced by the mixed gas only by using the heating element 61, without using the first temperature detector 62 of the microchip 8.

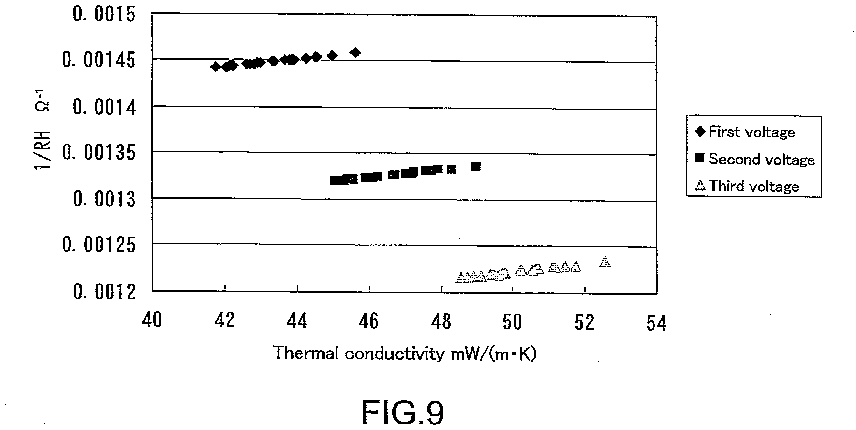

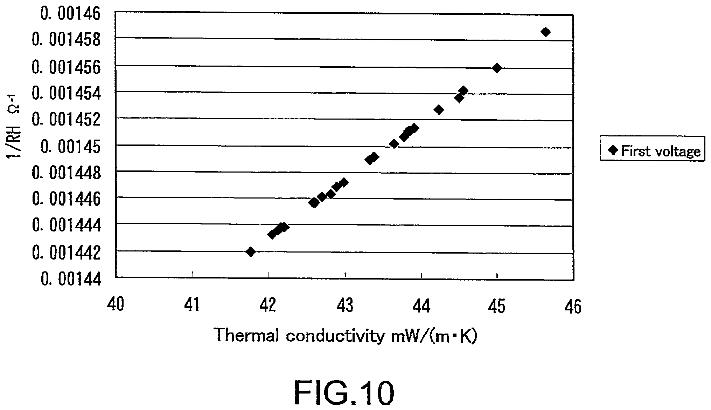

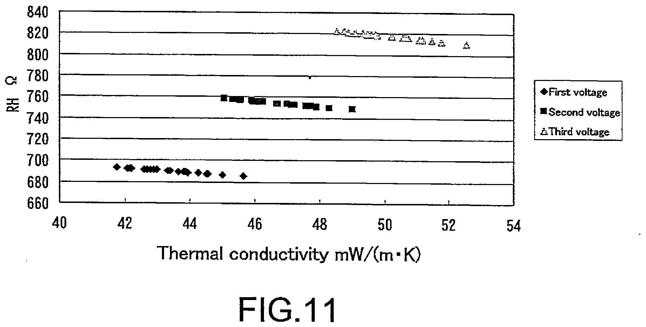

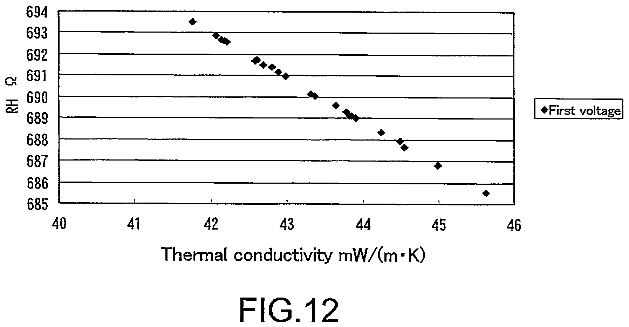

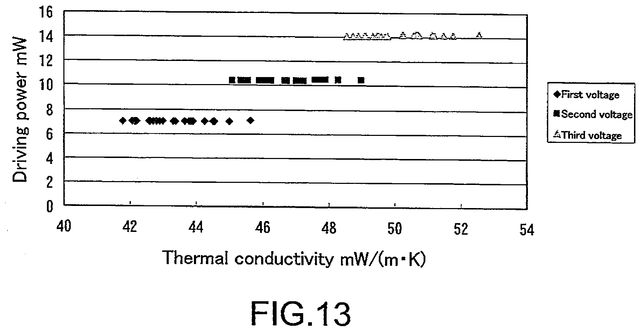

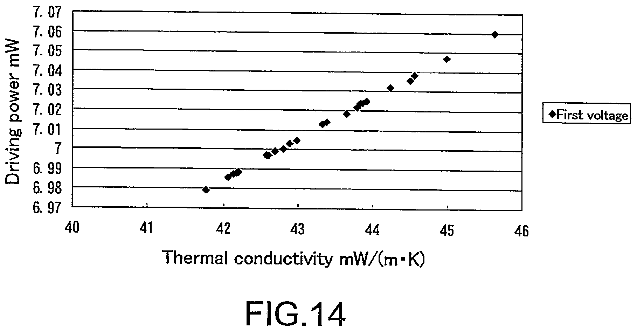

As shown in Equation (4), the heat-radiation coefficient $M_I$ of gas is in proportion to $1/R_H$. $1/R_H$ is the inverse number of the resistance value $R_H$ of the heating element 61. Further, as described above, the heat-radiation coefficient is in proportion to the thermal conductivity. As a result, the inverse number $(1/R_H)$ of the resistance value $R_H$ of the heating element 61 is in proportion to the thermal conductivity. Fig. 9 is a graph showing relation between the thermal conductivity and the inverse number $(1/R_H)$ of the resistance value $R_H$ of the heating element 61. In Fig. 9, the first voltage $V_1$, the second voltage $V_2$, and the third voltage $V_3$ are applied to the heating element 61. As shown in Fig. 9 and Fig. 10, the thermal conductivity is in proportion to the inverse number $(1/R_H)$ of the resistance value $R_H$ of the heating element 61, if the voltage applied to the heating element 61 is constant. Further, as shown in Fig. 11 and Fig. 12, the thermal conductivity is in correlation with the resistance value $R_H$ of the heating element 61 if the voltage applied to the heating element 61 is constant. Further, as shown in Fig. 13 and Fig. 14, the thermal conductivity is in correlation with the electric power for driving the heating element 61, if the voltage applied to the heating element 61 is constant.

In view of this, $1/R_{HA}$ is indicative of the inverse number of the resistance value $R_H$ of the heating element 61 in a case where the heating element 61 is exposed to the gas A. $1/R_{HB}$ is indicative of the inverse number of the resistance value $R_H$ of the heating element 61 in a case where the heating element 61 is exposed to the gas B. $1/R_{HC}$ is indicative of the inverse number of the resistance value $R_H$ of the heating element 61 in a case where the heating element 61 is exposed to the gas C. $1/R_{HD}$ is indicative of the inverse number of the resistance value $R_H$ of the heating element 61 in a case where the heating element 61 is exposed to the gas D. In this case, the inverse number ($1/R_{HI}$) of the resistance value $R_H$ of the heating element 61 exposed to the mixed gas is obtained by modifying Equation (12). That is, $1/R_{HI}$ equals to the sum of values, which are obtained by multiplying the volume fractions of the gas components by the inverse numbers of the resistance values $R_H$ in a case where the heating element 61 is exposed to the gas components, respectively. As a result, the following Equation (24) shows the inverse number ($1/R_{HI}$) of the resistance value $R_H$ of the heating element 61 exposed to the mixed gas to which a constant voltage is applied.

$$1/R_{HI}=1/R_{HA}\times V_A+1/R_{HB}\times V_B+1/R_{HC}\times V_C+1/R_{HD}\times V_D \ \ \cdots(24)$$

Further, the resistance value $R_H$ of the heating element 61 depends on the temperature $T_H$ of the heating element 61. So the inverse number ($1/R_{HI}$) of the resistance value $R_H$ of the heating element 61 exposed to the mixed gas is obtained based on the following Equation (25) as a function of the temperature $T_H$ of the heating element 61.

$$1/R_{HI}(T_H)=1/R_{HA}(T_H)\times V_A+1/R_{HB}(T_H)\times V_B+1/R_{HC}(T_H)\times V_C+1/R_{HD}(T_H)\times V_D \ \ \cdots(25)$$

In view of this, the following Equation (26) shows the inverse number ($1/R_{HI1}$) of the resistance value $R_H$ of the heating element 61 exposed to the mixed gas in a case where the temperature of the heating element 61 is $T_{H1}$. Further, the following Equation (27) shows the inverse number ($1/R_{HI2}$) of the resistance value $R_H$ of the heating element 61 exposed to the mixed gas in a case where the temperature of the heating element 61 is $T_{H2}$. The following Equation (28) shows the inverse number ($1/R_{HI3}$) of the resistance value $R_H$ of the heating element 61 exposed to the mixed gas in a case where the temperature of the heating element 61 is $T_{H3}$.

$$1/R_{HI1}(T_{H1})=1/R_{HA}(T_{H1})\times V_A+1/R_{HB}(T_{H1})\times V_B+1/R_{HC}(T_{H1})\times V_C+1/R_{HD}(T_{H1})\times V_D \ \ \cdots(26)$$

$$1/R_{HI2}(T_{H2})=1/R_{HA}(T_{H2})\times V_A+1/R_{HB}(T_{H2})\times V_B+1/R_{HC}(T_{H2})\times V_C+1/R_{HD}(T_{H2})\times V_D \ \ \cdots(27)$$

$$1/R_{HI3}(T_{H3})=1/R_{HA}(T_{H3})\times V_A+1/R_{HB}(T_{H3})\times V_B+1/R_{HC}(T_{H3})\times V_C+1/R_{HD}(T_{H3})\times V_D \ \ \cdots(28)$$

In Equation (26) to Equation (28), the resistance values $R_{HA}(T_{H1})$, $R_{HB}(T_{H1})$, $R_{HC}(T_{H1})$, $R_{HD}(T_{H1})$, $R_{HA}(T_{H2})$, $R_{HB}(T_{H2})$, $R_{HC}(T_{H2})$, $R_{HD}(T_{H2})$, $R_{HA}(T_{H3})$, $R_{HB}(T_{H3})$, $R_{HC}(T_{H3})$, $R_{HD}(T_{H3})$ of the heating element 61 exposed to the respective gas components may be previously obtained by measurement or the like. In this case, the following Equations (29) to (32) are obtained by solving a simultaneous equation including Equation (10) and Equations (26) to (28). Equations (29) to (32) show the volume fraction $V_A$ of the gas A, the volume fraction $V_B$ of the gas B, the volume fraction $V_C$ of the gas C, and the volume fraction $V_D$ of the gas, respectively. Equations (29) to (32) are functions of the resistance values $R_{HI1}(T_{H1})$, $R_{HI2}(T_{H2})$, $R_{HI3}(T_{H3})$ of the heating element 61 exposed to the mixed gas. Note that, in the following Equations (29) to (32), n is a natural number. $f_n$ is indicative of a function.

$$V_A=f_5[1/R_{HI1}(T_{H1}),\ 1/R_{HI2}(T_{H2}),\ 1/R_{HI3}(T_{H3})] \ \cdots(29)$$

$$V_B=f_6[1/R_{HI1}(T_{H1}),\ 1/R_{HI2}(T_{H2}),\ 1/R_{HI3}(T_{H3})] \ \cdots(30)$$

$$V_C=f_7[1/R_{HI1}(T_{H1}),\ 1/R_{HI2}(T_{H2}),\ 1/R_{HI3}(T_{H3})] \ \cdots(31)$$

$$V_D=f_8[1/R_{HI1}(T_{H1}),\ 1/R_{HI2}(T_{H2}),\ 1/R_{HI3}(T_{H3})] \ \cdots(32)$$

Here, Equations (29) to (32) are substituted in Equation (11). As a result, the following Equation (33) is obtained.

$$Q=K_A \times V_A+K_B \times V_B+K_C \times V_C+K_D \times V_D$$
$$=K_A \times f_5[1/R_{HI1}(T_{H1}), \ 1/R_{HI2}(T_{H2}), \ 1/R_{HI3}(T_{H3})]$$
$$+K_B \times f_6[1/R_{HI1}(T_{H1}), \ 1/R_{HI2}(T_{H2}), \ 1/R_{HI3}(T_{H3})]$$
$$+K_C \times f_7[1/R_{HI1}(T_{H1}), \ 1/R_{HI2}(T_{H2}), \ 1/R_{HI3}(T_{H3})]$$
$$+K_D \times f_8[1/R_{HI1}(T_{H1}), \ 1/R_{HI2}(T_{H2}), \ 1/R_{HI3}(T_{H3})] \ ...(33)$$

As shown in Equation (33), the calorific value Q of the mixed gas per unit volume is obtained based on an equation in which the resistance values $R_{HI1}(T_{H1})$, $R_{HI2}(T_{H2})$, $R_{HI3}(T_{H3})$ of the heating element 61 in the case where the temperatures of the heating element 61 are $T_{H1}$, $T_{H2}$, and $T_{H3}$, respectively, are variables. In view of this, the calorific value Q of the mixed gas is obtained based on the following Equation (34). Each of $g_2$ and $g_3$ is indicative of a function.

$$Q=g_2[1/R_{HI1}(T_{H1}), \ 1/R_{HI2}(T_{H2}), \ 1/R_{HI3}(T_{H3})]$$
$$=g_3[R_{HI1}(T_{H1}), \ R_{HI2}(T_{H2}), \ R_{HI3}(T_{H3})] \ ...(34)$$

In view of this, the inventors have found out the following fact. That is, it is possible to easily calculate the calorific value Q produced by the measuring-target mixed gas, per unit volume, by previously obtaining Equation (34) about the mixed gas including the gas A, the gas B, the gas C, and the gas D, even if the volume fraction $V_A$ of the gas A, the volume fraction $V_B$ of the gas B, the volume fraction $V_C$ of the gas C, and the volume fraction $V_D$ of the gas D are unknown. Specifically, the resistance values $R_{HI1}(T_{H1})$, $R_{HI2}(T_{H2})$, $R_{HI3}(T_{H3})$ of the heating element 61 in the case where the heating temperatures of the heating element 61 are $T_{H1}$, $T_{H2}$, and $T_{H3}$, respectively, are measured. The measured $R_{HI1}(T_{H1})$, $R_{HI2}(T_{H2})$, $R_{HI3}(T_{H3})$ are substituted in Equation (34). As a result, it is possible to uniquely obtain the calorific value Q produced by the measuring-target mixed gas, per unit volume. Further, in this case, it is possible to obtain the calorific value Q produced by the mixed gas, per unit volume, only by using the heating element 61, without using the first temperature detector 62 of the microchip 8.

Further, the resistance R is in correlation with the current I. So the calorific value Q produced by the mixed gas, per unit volume, is obtained based on the following Equation (35). In Equation (35), $g_4$ is indicative of a function. The currents $I_{H1}(T_{H1})$, $I_{H2}(T_{H2})$, $I_{H3}(T_{H3})$ passing through the heating element 61 in the case where the temperatures of the heating element 61 are $T_{H1}$, $T_{H2}$, and $T_{H3}$, respectively, are variables.

$$Q=g_4[I_{H1}(T_{H1}), \ I_{H2}(T_{H2}), \ I_{H3}(T_{H3})] \ ...(35)$$

Further, the resistance R of the heating element 61 is in correlation with the output signal AD output from the analog-digital converter circuit (hereinafter referred to as "A/D converter circuit") connected to the heating element 61. So the calorific value Q produced by the mixed gas, per unit volume, is obtained based on the following Equation (36). In Equation (36), $g_5$ is indicative of a function. The output signals $AD_{H1}(T_{H1})$, $AD_{H2}(T_{H2})$, $AD_{H3}(T_{H3})$ output from the A/D converter circuit in the case where the temperatures of the heating element 61 are $T_{H1}$, $T_{H2}$, and $T_{H3}$, respectively, are variables.

$$Q=g_5[AD_{H1}(T_{H1}), \ AD_{H2}(T_{H2}), \ AD_{H3}(T_{H3})] \ ...(36)$$

As a result, the calorific value Q produced by the mixed gas, per unit volume, is based on the following Equation (37). $g_6$ is indicative of a function. The electric signals $S_{H1}(T_{H1})$, $S_{H2}(T_{H2})$, $S_{H3}(T_{H3})$ output from the heating element 61 in the case where the heating temperatures of the heating element 61 are $T_{H1}$, $T_{H2}$, and $T_{H3}$, respectively, are variables.

$$Q=g_6[S_{H1}(T_{H1}), \ S_{H2}(T_{H2}), \ S_{H3}(T_{H3})] \ ...(37)$$

Note that the number of the kinds of gas components in the mixed gas may not be limited to four. For example, in a case where the mixed gas includes n kinds of gas components, the following Equation (38) is previously obtained. In Equation (38), $g_7$ is indicative of a function. The electric signals $S_{H1}(T_{H1})$, $S_{H2}(T_{H2})$, $S_{H3}(T_{H3})$, ..., $S_{Hn-1}(T_{Hn-1})$ output from the heating element 61 at at least n-1 kinds of heating temperatures $T_{H1}$, $T_{H2}$, $T_{H3}$, ..., $T_{Hn-1}$, respectively, are variables. Then, the electric signals $S_{H1}(T_{H1})$, $S_{H2}(T_{H2})$, $S_{H3}(T_{H3})$, ..., $S_{Hn-1}(T_{Hn-1})$ output from the heating element 61 at the n-1 kinds of heating temperatures $T_{H1}$, $T_{H2}$, $T_{H3}$, ..., $T_{Hn-1}$ are measured, respectively. Here, the heating element 61 is exposed to the measuring-target mixed gas including n kinds of gas components, the volume fractions of the n kinds of gas components being unknown. The measured $S_{H1}(T_{H1})$, $S_{H2}(T_{H2})$, $S_{H3}(T_{H3})$, ..., $S_{Hn-1}(T_{Hn-1})$ are substituted in Equation (38). As a result, it is possible to uniquely obtain the calorific value Q produced by the measuring-target mixed gas, per unit volume.

$$Q=g_7 [S_{H1}(T_{H1}), \ S_{H2}(T_{H2}), \ S_{H3}(T_{H3}), \ ..., \ S_{Hn-1}(T_{Hn-1})] \ ...(38)$$

Note that the mixed gas includes methane ($CH_4$), propane ($C_3H_8$), and in addition alkane ($C_jH_{2j+2}$) other than methane ($CH_4$) and propane ($C_3H_8$) as gas components, where j is a natural number. In this case, even if the alkane ($C_jH_{2j+2}$) other than methane ($CH_4$) and propane ($C_3H_8$) is considered as a mixture of methane ($CH_4$) and propane ($C_3H_8$), it does not affect calculation of Equation (38). For example, as shown in the following Equations (39) to (42), ethane ($C_2H_6$), butane ($C_4H_{10}$), pentane ($C_5H_{12}$), or hexane ($C_6H_{14}$) may be considered as a mixture of methane ($CH_4$) and propane ($C_3H_8$), the mixture being multiplied by a predetermined coefficient, and Equation (38) may be calculated.

$$C_2H_6=0.5CH_4+0.5C_3H_8 \ ...(39)$$

$$C_4H_{10}=-0.5CH_4+1.5C_3H_8 \ ...(40)$$

$$C_5H_{12}=-1.0CH_4+2.0C_3H_8 \ ...(41)$$

$$C_6H_{14}=-1.5CH_4+2.5C_3H_8 \ ...(42)$$

The mixed gas, which includes n kinds of gas components, includes methane ($CH_4$), propane ($C_3H_8$), and in addition z kinds of alkane ($C_jH_{2j+2}$) other than methane ($CH_4$) and propane ($C_3H_8$) as the gas components, where z is a natural number. In this case, an equation, in which electric signals $S_H$ output from the heating element 61 at at least n-z-1 kinds of heating temperatures are variables, may thus be obtained.

Note that, as a matter of course, it is possible to use Equation (38) in a case where the kinds of gas components in the mixed gas used for calculation of Equation (38) are the same as the kinds of gas components exposed to the measuring-target mixed gas, whose calorific value Q per unit volume is unknown. As a result, the calorific value Q produced by the measuring-target mixed gas, per unit volume, is calculated. Further, it is also possible to use Equation (38) in a case where the number of kinds of gas components exposed to the measuring-target mixed gas is less than n, and where the number of gas components in the mixed gas used for calculation of Equation (38) is less than n. For example, the mixed gas used for calculation of Equation (38) includes four kinds of gas components, i.e., methane ($CH_4$), propane ($C_3H_8$), nitrogen ($N_2$), and carbon dioxide ($CO_2$). In this case, it is also possible to use Equation (38) in a case where the measuring-target mixed gas does not include nitrogen ($N_2$), but only includes three kinds of gas components, i.e., methane ($CH_4$), propane ($C_3H_8$), and carbon dioxide ($CO_2$). As a result, the calorific value Q produced by the measuring-target mixed gas, per unit volume, is calculated.

Further, the mixed gas used for calculation of Equation (38) includes methane ($CH_4$) and propane ($C_3H_8$) as gas components. In this case, it is possible to use Equation (38) in a case where the measuring-target mixed gas includes alkane ($C_jH_{2j+2}$), which is not included in the mixed gas used for calculation of Equation (38). The reason is as follows. That is, as described above, alkane ($C_jH_{2j+2}$) other than methane ($CH_4$) and propane ($C_3H_8$) may be considered as a mixture of methane ($CH_4$) and propane ($C_3H_8$). It does not affect calculation of the calorific value Q per unit volume by using Equation (38).

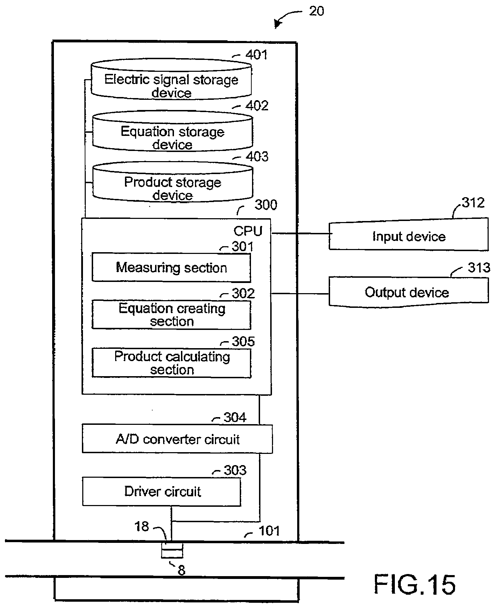

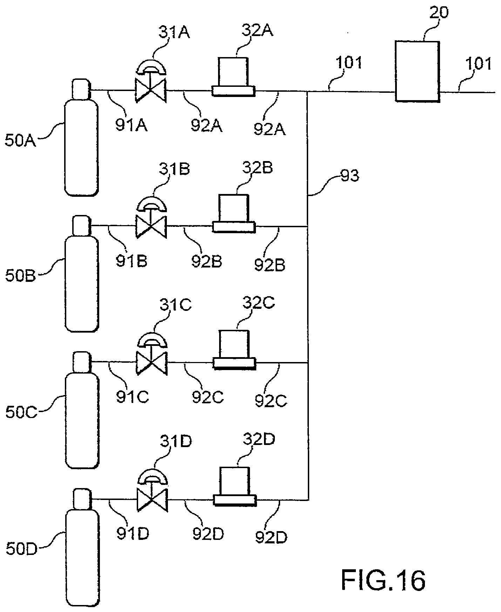

Here, each of Fig. 15 and Fig. 16 shows a calorific-value measuring system 20 according to the embodiment. The gas measuring system 20 includes a pipe 101 and the microchip 8 of Fig. 1 or Fig. 3. Each of a plurality of kinds of sample mixed gas flows in the pipe 101. The microchip 8 is arranged in the pipe 101. The microchip 8 includes the heating element 61. The heating element 61 produces heat at a plurality of heating temperatures $T_H$. Hereinafter, an example in which the calorific-value measuring system 20 includes the microchip 8 of Fig. 1 will be described. Alternatively, the calorific-value measuring system 20 may include the microchip 8 of Fig. 3. Behaviors (Fig. 15) of the calorific-value measuring system 20 including the microchip 8 of Fig. 1 are similar to behaviors of the calorific-value measuring system 20 including the microchip 8 of Fig. 3.

The microchip 8 is arranged on the pipe 101 via the heat-insulating member 18, and projects in the pipe 101. Note that part of the microchip 8 may be embedded in the side wall of the pipe 101 as long as at least the heating element 61 of Fig. 1 projects in the pipe 101 of Fig. 15. Since the microchip 8 is arranged such that the microchip 8 projects in the pipe 101, gas in the cavity 66 of the microchip 8 of Fig. 2 is exchanged rapidly. As a result, the microchip 8 is more responsive.

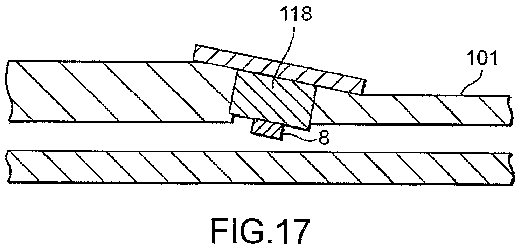

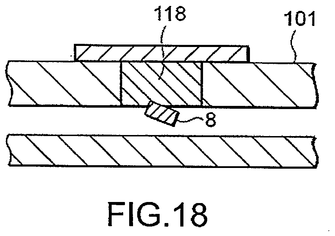

Further, as shown in Fig. 17, the entire package 118 including the microchip 8 may be arranged such that the package 118 is oblique to the side wall of the pipe 101. Alternatively, as shown in Fig. 18, the microchip 8 is obliquely die-bonded to the package 118, and the microchip 8 is thus oblique to the side wall of the pipe 101. By arranging the microchip 8 obliquely to the side wall of the pipe 101, the microchip 8 faces gas flowing in the pipe 101. As a result, gas in the cavity 66 of the microchip 8 of Fig. 2 is exchanged rapidly.

The calorific-value measuring system 20 further includes a measuring section 301 and an equation creating section 302. The measuring section 301 measures values of the electric signals $S_H$ output from the heating element 61. The heating element 61 is exposed to each of the plurality of kinds of sample mixed gas, and produces heat at a plurality of heating temperatures $T_H$. The equation creating section 302 creates a calorific-value equation based on known calorific values Q of a plurality of kinds of sample mixed gas, and based on values of the electric signals $S_H$ output from the heating element 61 at a plurality of heating temperatures $T_H$, respectively. The calorific-value equation includes the electric signals $S_H$ output from the heating element 61 at a plurality of heating temperatures $T_H$, respectively, as independent variables. The calorific-value equation further includes the calorific value Q as a dependent variable. Note that each sample mixed gas includes a plurality of kinds of gas components.

Four kinds of sample mixed gas are used. The calorific value Q of one kind of sample mixed gas is different from the calorific value Q of any other kind of sample mixed gas. In this case, as shown in Fig. 16, a first gas cylinder 50A, a second gas cylinder 50B, a third gas cylinder 50C, and a fourth gas cylinder 50D are prepared. The first gas cylinder 50A stores a first sample mixed gas. The second gas cylinder 50B stores a second sample mixed gas. The third gas cylinder 50C stores a third sample mixed gas. The fourth gas cylinder 50D stores a fourth sample mixed gas. A first gas-pressure regulator 31A is connected to the first gas cylinder 50A via a pipe 91A. The first gas-pressure regulator 31A is capable of obtaining a first sample mixed gas from the first gas cylinder 50A, which is regulated at low pressure (e.g., 0.2 MPa). Further, a first flow controller 32A is connected to the first gas-pressure regulator 31A via a pipe 92A. The first flow controller 32A controls a flow rate of the first sample mixed gas, which is sent to the calorific-value measuring system 20 via the pipe 92A and the pipe 101.

A second gas-pressure regulator 31B is connected to the second gas cylinder 50B via a pipe 91B. Further, a second flow controller 32B is connected to the second gas-pressure regulator 31B via a pipe 92B. The second flow controller 32B controls a flow rate of the second sample mixed gas, which is sent to the calorific-value measuring system 20 via the pipes 92B, 93, 101.

A third gas-pressure regulator 31C is connected to the third gas cylinder 50C via a pipe 91C. Further, a third flow controller 32C is connected to the third gas-pressure regulator 31C via a pipe 92C. The third flow controller 32C controls a flow rate of the third sample mixed gas, which is sent to the calorific-value measuring system 20 via the pipes 92C, 93, 101.

A fourth gas-pressure regulator 31D is connected to the fourth gas cylinder 50D via a pipe 91D. Further, a fourth flow controller 32D is connected to the fourth gas-pressure regulator 31D via a pipe 92D. The fourth flow controller 32D controls a flow rate of the fourth sample mixed gas, which is sent to the calorific-value measuring system 20 via the pipes 92D, 93, 101.

Each of the first sample mixed gas to the fourth sample mixed gas is, for example, natural gas. Each of the first sample mixed gas to the fourth sample mixed gas includes four kinds of gas components (for example, methane ($CH_4$), propane ($C_3H_8$), nitrogen ($N_2$), and carbon dioxide ($CO_2$)).

The first sample mixed gas flows in the pipe 101 of Fig. 15 without stopping. In this situation, a driver circuit 303 of Fig. 15 sequentially provides drive powers $P_{H1}$, $P_{H2}$, $P_{H3}$ to the heating element 61 of Fig. 1 and Fig. 2. In a case where the drive powers $P_{H1}$, $P_{H2}$, $P_{H3}$ are supplied to the heating element 61, the heating element 61 exposed to the first sample mixed gas, which flows in the pipe 101 without stopping, produces heat at a temperature $T_{H1}$ (100°C), a temperature $T_{H2}$ (150°C), and a temperature $T_{H3}$ (200°C), for example. The heating element 61 outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$.

The first sample mixed gas is removed from the pipe 101. After that, the second sample mixed gas to the fourth sample mixed gas sequentially flow in the pipe 101. In a case where the second sample mixed gas flows in the pipe 101 without stopping, the heating element 61 of Fig. 1 and Fig. 2 outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$. The third sample mixed gas flows in the pipe 101 of Fig. 15 without stopping. In this case, the heating element 61 of Fig. 1 and Fig. 2 outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$. The fourth sample mixed gas flows in the pipe 101 of Fig. 15 without stopping. In this case, the heating element 61 of Fig. 1 and Fig. 2 outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$.

Note that each sample mixed gas includes n kinds of gas components. In this case, the heating element 61 of the microchip 8 of Fig. 1 and Fig. 2 produces heat at at least n-1 kinds of different temperatures. Note that, as described above, alkane $(C_jH_{2j+2})$ other than methane $(CH_4)$ and propane $(C_3H_8)$ may be considered as a mixture of methane $(CH_4)$ and propane $(C_3H_8)$. The sample mixed gas, which includes n kinds of gas components, includes methane $(CH_4)$, propane $(C_3H_8)$, and in addition z kinds of alkane $(C_jH_{2j+2})$ as gas components, where z is a natural number. In this case, the heating element 61 thus produces heat at at least n-z-1 kinds of different temperatures.

As shown in Fig. 15, the microchip 8 is connected to a central processing unit (CPU) 300. The CPU 300 includes the measuring section 301. An electric signal storage device 401 is connected to the CPU 300. The measuring section 301 measures values of the electric signal $S_{H1}(T_{H1})$ at the heating temperature $T_{H1}$, the electric signal $S_{H2}(T_{H2})$ at the heating temperature $T_{H2}$, and the electric signal $S_{H3}(T_{H3})$ at the heating temperature $T_{H3}$, which are output from the heating element 61. The measuring section 301 stores the measured values in the electric signal storage device 401. Note that the electric signal $S_H$ output from the heating element 61 is any one of the resistance value $R_H$ of the heating element 61, the current $I_H$ passing through the heating element 61, and an output signal $AD_H$ output from the A/D converter circuit 304 connected to the heating element 61.

The equation creating section 302 of the CPU 300 collects, for example, the known calorific values Q of the first sample mixed gas to the fourth sample mixed gas, respectively, and the plurality of measured values of the electric signals $S_{H1}(T_{H1})$, $S_{H2}(T_{H2})$, $S_{H3}(T_{H3})$ output from the heating element 61. Further, the equation creating section 302 calculates a calorific-value equation based on the collected calorific values Q and based on the collected values of the electric signals $S_H$ by means of multivariate statistics. The calorific-value equation includes the electric signals $S_{H1}(T_{H1})$, $S_{H2}(T_{H2})$, $S_{H3}(T_{H3})$ output from the heating element 61, as independent variables. The calorific-value equation further includes the calorific value Q as a dependent variable.

Note that, examples of "multivariate statistics" include support vector regression and multiple regression analysis disclosed in "A Tutorial on Support Vector Regression" (NeuroCOLT Technical Report (NC-TR-98-030), 1998) by A. J Smola and B. Scholkopf and fuzzy quantification theory of second kind disclosed in Japanese Patent Application Laid-open No. H05-141999.

The calorific-value measuring system 20 further includes equation storage device 402 connected to the CPU 300. The equation storage device 402 stores the calorific-value equation created by the equation creating section 302. Further, an input device 312 and an output device 313 are connected to the CPU 300. Examples of the input device 312 include a keyboard and a pointing device such as a mouse. Examples of the output device 313 include a printer and an image display device such as a liquid crystal display or a monitor.

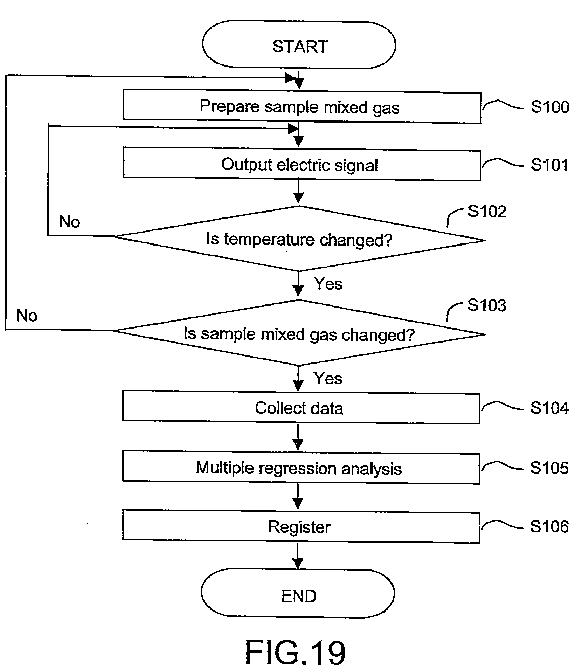

Next, with reference to a flowchart of Fig. 19, a calorific-value-equation creating method according to the embodiment will be described.

(a) In Step S100, valves of the second to fourth flow controllers 32B to 32D of Fig. 16 are closed, and a valve of the first flow controller 32A is open. The first sample mixed gas is introduced in the pipe 101 of Fig. 15. In Step S101, the driver circuit 303 supplies drive power $P_{H1}$ to the heating element 61 of Fig. 1 and Fig. 2. As a result, the heating element 61 produces heat at 100°C. The measuring section 301 of Fig. 15 measures a value of an electric signal $S_{H1}(T_{H1})$ output from the heating element 61, which is exposed to the first sample mixed gas flowing in the pipe 101 without stopping and produces heat at 100°C, and stores the value of the electric signal $S_{H1}(T_{H1})$ in the electric signal storage device 401.

(b) In Step S102, the driver circuit 303 determines whether the temperature of the heating element 61 of Fig. 1 and Fig. 2 is changed. If the temperature of the heating element 61 is not changed to 150°C and 200°C, Step S101 is performed again. The driver circuit 303 of Fig. 15 causes the heating element 61 of Fig. 1 and Fig. 2 to produce heat at 150°C. The measuring section 301 of Fig. 15 measures a value of an electric signal $S_{H2}(T_{H2})$ output from the heating element 61, which is exposed to the first sample mixed gas flowing in the pipe 101 without stopping and produces heat at 150°C, and stores the value of the electric signal $S_{H2}(T_{H2})$ in the electric signal storage device 401.

(c) In Step S102, the driver circuit 303 determines whether the temperature of the heating element 61 of Fig. 1 and Fig. 2 is changed again. If the temperature of the heating element 61 is not changed to 200°C, Step S101 is performed again. The driver circuit 303 of Fig. 15 causes the heating element 61 of Fig. 1 and Fig. 2 to produce heat at 200°C. The measuring section 301 of Fig. 15 receives an electric signal $S_{H3}(T_{H3})$ output from the heating element 61, which is exposed to the first sample mixed gas flowing in the pipe 101 without stopping and produces heat at 200°C, and stores the value of the electric signal $S_{H3}(T_{H3})$ in the electric signal storage device 401.

(d) If the temperature of the heating element 61 is changed, Step S102 is finished and Step S103 is performed. In Step S103, whether the sample mixed gas is changed is determined. If the sample mixed gas is not changed to the second sample mixed gas to the fourth sample mixed gas, Step S100 is performed again. In Step S100, the first flow controller 32A of Fig. 16 is closed, the valves of the third to fourth flow controllers 32C to 32D are closed, and the valve of the second flow controller 32B is open. The second sample mixed gas is introduced in the pipe 101 of Fig. 15.

(e) Similar to the case of the first sample mixed gas, the loop of Step S101 and Step S102 is repeated. The heating element 61 is exposed to the second sample mixed gas flowing in the pipe 101 without stopping, and produces heat at 100°C, 150°C, and 200°C. The measuring section 301 measures values of the electric signals $S_{H1}(T_{H1})$, $S_{H2}(T_{H2})$, $S_{H3}(T_{H3})$ output from the heating element 61. The measuring section 301 stores the values of the electric signals $S_{H1}(T_{H1})$, $S_{H2}(T_{H2})$, $S_{H3}(T_{H3})$ in the electric signal storage device 401.

(f) After that, the loop of Step S100 to Step S103 is repeated. The heating element 61 is exposed to the third sample mixed gas flowing in the pipe 101 without stopping. The heating element 61 produces heat at 100°C, 150°C, and 200°C. Values of the electric signals $S_{H1}(T_{H1})$, $S_{H2}(T_{H2})$, $S_{H3}(T_{H3})$ output from the heating element 61 are thus stored in the electric signal storage device 401. Further, the heating element 61 is exposed to the fourth sample mixed gas flowing in the pipe 101 without stopping. The heating element 61 produces heat at 100°C, 150°C, and 200°C. Values of the electric signals $S_{H1}(T_{H1})$, $S_{H2}(T_{H2})$, $S_{H3}(T_{H3})$ output from the heating element 61 are thus stored in the electric signal storage device 401.

(g) In Step S104, the input device 312 inputs, in the equation creating section 302, the known calorific value Q of the first sample mixed gas, the known calorific value Q of the second sample mixed gas, the known calorific value Q of the third sample mixed gas, and the known calorific value Q of the fourth sample mixed gas. Further, the equation creating section 302 retrieves the plurality of measured values of the electric signals $S_{H1}(T_{H1})$, $S_{H2}(T_{H2})$, $S_{H3}(T_{H3})$ output from the heating element 61, from the electric signal storage device 401.

(h) In Step S105, the equation creating section 302 performs multiple regression analysis based on the calorific values Q of the first sample mixed gas to the fourth sample mixed gas, and based on the plurality of measured values of the electric signals $S_{H1}(T_{H1})$, $S_{H2}(T_{H2})$, $S_{H3}(T_{H3})$ output from the heating element 61. As the result of multiple regression analysis, the equation creating section 302 calculates a calorific-value equation including the electric signals $S_{H1}(T_{H1})$, $S_{H2}(T_{H2})$, $S_{H3}(T_{H3})$ output from the heating element 61 as independent variables, and the calorific value Q as a dependent variable. After that, in Step S106, the equation creating section 302 stores the created calorific-value equation in the equation storage device 402. The calorific-value-equation creating method according to the embodiment is thus finished.

As described above, according to the calorific-value-equation creating method of the embodiment, it is possible to create a calorific-value equation capable of uniquely calculating the calorific value Q of the measuring-target mixed gas.

Next, functions of the calorific-value measuring system 20 according to the embodiment in the case of measuring the calorific value Q of the measuring-target mixed gas whose calorific value Q is unknown will be described. The measuring-target mixed gas such as natural gas is introduced in the pipe 101 via a flow controller. For example, the measuring-target mixed gas includes methane ($CH_4$), propane ($C_3H_8$), nitrogen ($N_2$), carbon dioxide ($CO_2$), and the like of unknown volume fractions. The calorific value Q of the measuring-target mixed gas is unknown. The driver circuit 303 of Fig. 15 sequentially supplies the drive powers $P_{H1}$, $P_{H2}$, $P_{H3}$ to the heating element 61 of the microchip 8 of Fig. 1 and Fig. 2. In the case where the drive powers $P_{H1}$, $P_{H2}$, $P_{H3}$ are supplied to the heating element 61, the heating element 61 exposed to the measuring-target mixed gas, which flows in the pipe 101 without stopping, sequentially produces heat at the temperature $T_{H1}$ (100°C), the temperature $T_{H2}$ (150°C), and the temperature $T_{H3}$ (200°C), for example. The heating element 61 outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$.

The measuring section 301 of Fig. 15 measures values of the electric signal $S_{H1}(T_{H1})$ at the heating temperature $T_{H1}$, the electric signal $S_{H2}(T_{H2})$ at the heating temperature $T_{H2}$, and the electric signal $S_{H3}(T_{H3})$ at the heating temperature $T_{H3}$, which are output from the heating element 61 exposed to the measuring-target mixed gas, which flows in the pipe 101 without stopping. The measuring section 301 stores the measured values in the electric signal storage device 401.

As described above, the equation storage device 402 stores a calorific-value equation including the electric signal $S_{H1}(T_{H1})$ output from the heating element 61 at the heating temperature $T_{H1}$ (100°C), the electric signal $S_{H2}(T_{H2})$ output from the heating element 61 at the heating temperature $T_{H2}$ (150°C), and the electric signal $S_{H3}(T_{H3})$ output from the heating element 61 at the heating temperature $T_{H3}$ (200°C), as independent variables, and further including the calorific value Q of the gas, as a dependent variable.

The calorific-value measuring system 20 according to the embodiment further includes a calorific-value calculating section 305. The calorific-value calculating section 305 substitutes the measured values of the electric signals $S_{H1}(T_{H1})$, $S_{H2}(T_{H2})$, $S_{H3}(T_{H3})$ output from the heating element 61 in the independent variables in the calorific-value equation. The independent variables indicate the electric signals $S_{H1}(T_{H1})$, $S_{H2}(T_{H2})$, $S_{H3}(T_{H3})$ output from the heating element 61. The calorific-value calculating section 305 calculates the measured value of the calorific value Q of the measuring-target mixed gas flowing in the pipe 101 without stopping. A calorific-value storage device 403 is further connected to the CPU 300. The calorific-value storage device 403 stores the calorific value Q of the measuring-target mixed gas calculated by the calorific-value calculating section 305.

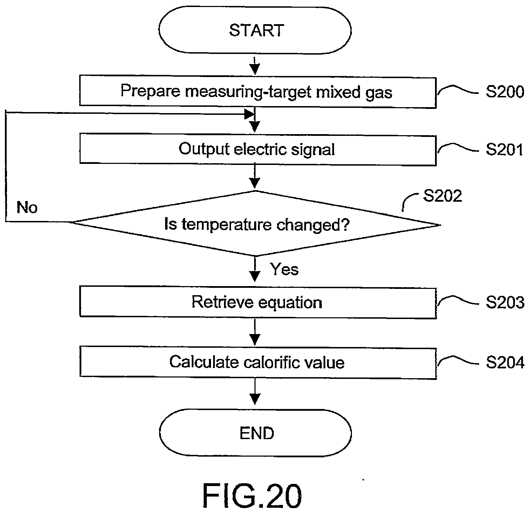

Next, with reference to a flowchart of Fig. 20, a calorific-value measuring method according to the embodiment will be described.

(a) In Step S200, the measuring-target mixed gas is introduced in the pipe 101 of Fig. 15. In Step S201, the driver circuit 303 supplies drive power $P_{H1}$ to the heating element 61 of Fig. 1 and Fig. 2. As a result, the heating element 61 produces heat at 100°C. The measuring section 301 of Fig. 15 receives an electric signal $S_{H1}(T_{H1})$ from the heating element 61, which is exposed to the measuring-target mixed gas flowing in the pipe 101 without stopping and produces heat at 100°C. The measuring section 301 stores the value of the electric signal $S_{H1}(T_{H1})$ in the electric signal storage device 401.

(b) In Step S202, the driver circuit 303 of Fig. 15 determines whether the temperature of the heating element 61 of Fig. 1 and Fig. 2 is changed. If the temperature of the heating element 61 is not changed to 150°C and 200°C, Step S201 is performed again. The driver circuit 303 supplies drive power $P_{H2}$ to the heating element 61 of Fig. 1 and Fig. 2 to thereby cause the heating element 61 of Fig. 1 and Fig. 2 to produce heat at 150°C. The measuring section 301 of Fig. 15 receives an electric signal $S_{H2}(T_{H2})$ output from the heating element 61, which is exposed to the measuring-target mixed gas flowing in the pipe 101 without stopping and produces heat at 150°C, and stores the value of the electric signal $S_{H2}(T_{H2})$ in the electric signal storage device 401.

(c) In Step S202, the driver circuit 303 determines whether the temperature of the heating element 61 of Fig. 1 and Fig. 2 is changed again. If the temperature of the heating element 61 is not changed to 200°C, Step S201 is performed again. The driver circuit 303 supplies drive power $P_{H3}$ to the heating element 61 of Fig. 1 and Fig. 2 to thereby cause the heating element 61 of Fig. 1 and Fig. 2 to produce heat at 200°C. The measuring section 301 of Fig. 15 receives an electric signal $S_{H3}(T_{H3})$ output from the heating element 61, which is exposed to the measuring-target mixed gas flowing in the pipe 101 without stopping and produces heat at 200°C, and stores the value of the electric signal $S_{H3}(T_{H3})$ in the electric signal storage device 401.

(d) If the temperature of the heating element 61 is changed, Step S202 is finished and Step S203 is performed. In Step S203, the calorific-value calculating section 305 of Fig. 15 retrieves, from the equation storage device 402, the calorific-value equation including the electric signals $S_{H1}(T_{H1})$, $S_{H2}(T_{H2})$, $S_{H3}(T_{H3})$ output from the heating element 61, as independent variables, and the calorific value Q as a dependent variable. Further, the calorific-value calculating section 305 retrieves, from the electric signal storage device 401, the measured values of the electric signals $S_{H1}(T_{H1})$, $S_{H2}(T_{H2})$, $S_{H3}(T_{H3})$ output from the heating element 61 exposed to the measuring-target mixed gas.

(e) In Step S204, the calorific-value calculating section 305 substitutes measured values in the independent variables in the calorific-value equation. The independent variables indicate the electric signals $S_{H1}(T_{H1})$, $S_{H2}(T_{H2})$, $S_{H3}(T_{H3})$. The calorific-value calculating section 305 calculates the calorific value Q of the measuring-target mixed gas. After that, the calorific-value calculating section 305 stores the calculated calorific value Q in the calorific-value storage device 403. The calorific-value measuring method according to the embodiment is thus finished.

As described above, according to the calorific-value calculating method of the embodiment, it is possible to measure the calorific value Q of the measuring-target mixed gas based on the values of the electric signals $S_{H1}(T_{H1})$, $S_{H2}(T_{H2})$, $S_{H3}(T_{H3})$ output from the heating element 61 exposed to the measuring-target mixed gas flowing in the pipe 101 without stopping. In addition, it is not necessary to use an expensive gas chromatograph or a sound-speed sensor to measure the calorific value Q of the measuring-target mixed gas.

Component fractions of carbon hydride in natural gas are different depending on a gas field from which the natural gas is yielded. Further, natural gas includes carbon hydride, in addition, nitrogen ($N_2$), carbon dioxide ($CO_2$), and the like. So volume fractions of gas components in natural gas are different depending on a gas field from which the natural gas is yielded. In view of this, even if the kinds of gas components are known, the calorific value Q of the natural gas is unknown, in most cases. Further, the calorific value Q of natural gas yielded from the same gas field is not necessarily the same. The calorific value Q may be different depending on a yielding season.

Conventionally, a natural-gas fee is charged not based on a calorific value Q of used natural gas, but based on volume of the used natural gas. However, since the calorific value Q of natural gas is different depending on a gas field from which the natural gas is yielded, it is unfair to charge on volume of the used natural gas. To the contrary, by using the calorific-value calculating method according to the embodiment, it is possible to easily calculate the calorific value Q of mixed gas such as natural gas whose kinds of gas components are known but whose calorific value Q is unknown because volume fractions of the gas components are unknown. As a result, it is possible to charge fair gas fees.

Further, in a case of driving a gas turbine, it is necessary to monitor, without delay, the calorific value Q of natural gas as fuel gas supplied to the gas turbine. The reason is as follows. In a case where the calorific value Q of fuel gas is not constant, the gas turbine may be broken because of combustion oscillation and the like. However, response time of a conventional calorimeter is large (several minutes). Such a conventional calorimeter is not suitable to control the calorific value Q of fuel gas supplied to a gas turbine. To the contrary, it takes several seconds for the calorific-value measuring system according to the embodiment to measure a calorific value. So the calorific-value measuring system according to the embodiment is also suitable to control the calorific value Q of fuel gas supplied to a gas turbine.

Further, according to the calorific-value calculating method of the embodiment, it is possible to easily and accurately obtain the calorific value Q of mixed gas such as natural gas. Because of this, it is possible to appropriately set volume of air necessary to combust mixed gas. As a result, it is also possible to reduce the amount of unnecessary carbon dioxide ($CO_2$) emissions.

(Example 1)

First, 40 kinds of sample mixed gas were prepared. The calorific values Q of the prepared 40 kinds of sample mixed gas were known. Each of the 40 kinds of sample mixed gas included, as gas components, at least one of or all of methane ($CH_4$), ethane ($C_2H_6$), propane ($C_3H_8$), butane ($C_4H_{10}$), nitrogen ($N_2$), and carbon dioxide ($CO_2$). For example, particular sample mixed gas included 90 vol% of methane, 3 vol% of ethane, 1 vol% of propane, 1 vol% of butane, 4 vol% of nitrogen, and 1 vol% of carbon dioxide. Further, particular sample mixed gas included 85 vol% of methane, 10 vol% of ethane, 3 vol% of propane, 2 vol% of butane, no nitrogen, and no carbon dioxide. Further, particular sample mixed gas included 85 vol% of methane, 8 vol% of ethane, 2 vol% of propane, 1 vol% of butane, 2 vol% of nitrogen, and 2 vol% of carbon dioxide.

Next, the 40 kinds of sample mixed gas were used, and a plurality of measured values of the electric signals $S_{H1}(T_{H1})$, $S_{H2}(T_{H2})$, $S_{H3}(T_{H3})$ output from the heating element were obtained. After that, a linear equation, a quadratic equation, and a cubic equation for calculating the calorific value Q were created by means of support vector regression based on the known calorific value Q of each of the 40 kinds of sample mixed gas, and based on the plurality of measured values of the electric signals $S_{H1}(T_{H1})$, $S_{H2}(T_{H2})$, $S_{H3}(T_{H3})$ output from the heating element. The electric signals $S_{H1}(T_{H1})$, $S_{H2}(T_{H2})$, $S_{H3}(T_{H3})$ output from the heating element were independent variables. The calorific value Q was a dependent variable.

In creating the linear equation for calculating the calorific value Q, the number of calibration points may be arbitrarily determined (e.g., 3 to 5). In creating the quadratic equation for calculating the calorific value Q, the number of calibration points may be arbitrarily determined (e.g., 8 to 9). In creating the cubic equation for calculating the calorific value Q, the number of calibration points may be arbitrarily determined (e.g., 10 to 14).

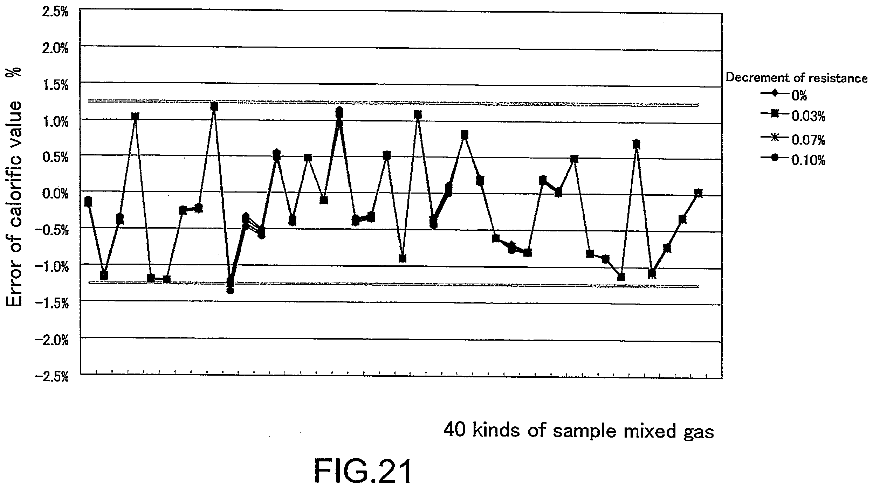

The calorific value Q of each of the 40 kinds of sample mixed gas was calculated by using the created calorific-value equations. The calculated calorific value Q was compared with the true calorific value Q. As shown in Fig. 21, an error was plus/minus 1.3% or less. Further, resistance of the heating element was decreased by 0.03%, 0.07%, and 0.10% on purpose. However, the error was not increased. It indicated that drift of the heating element due to aging degradation or the like did not affect calculation of a calorific value.

(Comparative Example)

As shown in Equation (9), the heat-radiation coefficient $M_I$ of mixed gas depends on the resistance value $R_H$ of the heating element and the resistance value $R_I$ of the temperature detector. In view of this, the calorific value Q of mixed gas per unit volume is obtained based on the following Equation (43). In Equation (43), the resistance values $R_{H1}(T_{H1})$, $R_{H2}(T_{H2})$, $R_{H3}(T_{H3})$ of the heating element in the case where the temperatures of the heating element are $T_{H1}$, $T_{H2}$, and $T_{H3}$, respectively, are variables. The resistance value $R_I$ of the temperature detector exposed to the mixed gas is a variable. g is indicative of a function.

$$Q = g[R_{H1}(T_{H1}),\ R_{H2}(T_{H2}),\ R_{H3}(T_{H3}),\ R_I]\ \ldots(43)$$

Further, the calorific value Q of mixed gas per unit volume is obtained based on the following Equation (44). In Equation (44), the currents $I_{H1}(T_{H1})$, $I_{H2}(T_{H2})$, $I_{H3}(T_{H3})$ passing through the heating element in the case where the temperatures of the heating element are $T_{H1}$, $T_{H2}$, and $T_{H3}$, respectively, are variables. The current $I_I$ of the temperature detector exposed to the mixed gas is a variable. g is indicative of a function.

$$Q = g[I_{H1}(T_{H1}),\ I_{H2}(T_{H2}),\ I_{H3}(T_{H3}),\ I_I]\ \ldots(44)$$

Alternatively, the calorific value Q of mixed gas per unit volume is obtained based on the following Equation (45). In Equation (45), the voltages $V_{H1}(T_{H1})$, $V_{H2}(T_{H2})$, $V_{H3}(T_{H3})$ applied to the heating element in the case where the temperatures of the heating element are $T_{H1}$, $T_{H2}$, and $T_{H3}$, respectively, are variables. The voltage $V_I$ applied to the temperature detector exposed to the mixed gas is a variable. g is indicative of a function.

$$Q=g[V_{H1}(T_{H1}),\ V_{H2}(T_{H2}),\ V_{H3}(T_{H3}),\ V_I]\ \ldots(45)$$

Alternatively, the calorific value Q of mixed gas per unit volume is obtained based on the following Equation (46). In Equation (46), the output signals $AD_{H1}(T_{H1})$, $AD_{H2}(T_{H2})$, $AD_{H3}(T_{H3})$ output from an analog-digital converter circuit (hereinafter referred to as "A/D converter circuit") connected to the heating element in the case where the temperatures of the heating element are $T_{H1}$, $T_{H2}$, and $T_{H3}$, respectively, are variables. The output signal $AD_I$ of an A/D converter circuit connected to the temperature detector exposed to the mixed gas is a variable. g is indicative of a function.

$$Q=g[AD_{H1}(T_{H1}),\ AD_{H2}(T_{H2}),\ AD_{H3}(T_{H3}),\ AD_I]\ \ldots(46)$$

In view of this, the calorific value Q of mixed gas per unit volume is obtained based on the following Equation (47). In Equation (47), the electric signals $S_{H1}(T_{H1})$, $S_{H2}(T_{H2})$, $S_{H3}(T_{H3})$ output from the heating element in the case where the heating temperatures of the heating element are $T_{H1}$, $T_{H2}$, and $T_{H3}$, respectively, are variables. The electric signal $S_I$ output from the temperature detector exposed to the mixed gas is a variable. g is indicative of a function.

$$Q=g[S_{H1}(T_{H1}),\ S_{H2}(T_{H2}),\ S_{H3}(T_{H3}),\ S_I]\ \ldots(47)$$

Here, the 40 kinds of sample mixed gas were used, which were the same as the 40 kinds of sample mixed gas of Example 1. A plurality of measured values of the electric signal $S_I$ output from the temperature detector, and a plurality of measured values of the electric signals $S_{H1}(T_{H1})$, $S_{H2}(T_{H2})$, $S_{H3}(T_{H3})$ output from the heating element were obtained. After that, a linear equation, a quadratic equation, and a cubic equation for calculating the calorific value Q were created by means of support vector regression based on the known calorific value Q of each of the 40 kinds of sample mixed gas, based on the plurality of measured values of the electric signal $S_I$ output from the temperature detector, and based on the plurality of measured values of the electric signals $S_{H1}(T_{H1})$, $S_{H2}(T_{H2})$, $S_{H3}(T_{H3})$ output from the heating element. The electric signal $S_I$ output from the temperature detector and the electric signals $S_{H1}(T_{H1})$, $S_{H2}(T_{H2})$, $S_{H3}(T_{H3})$ output from the heating element were independent variables. The calorific value Q was a dependent variable.

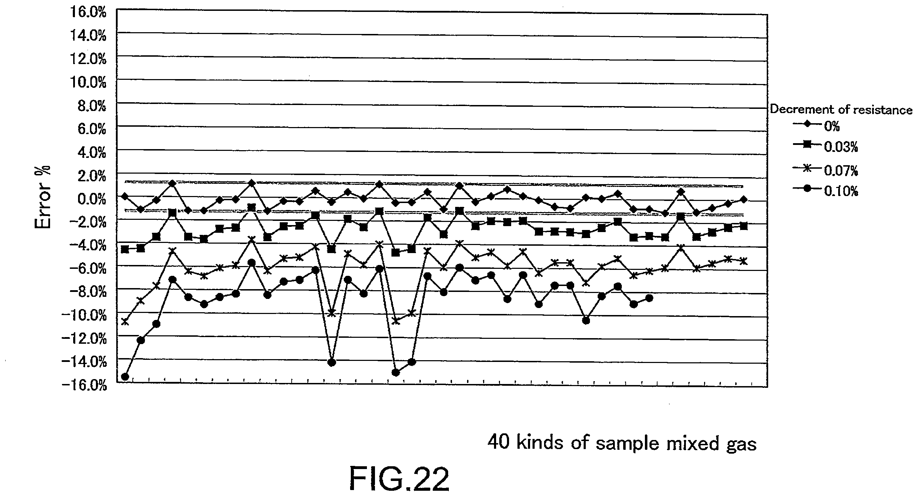

The calorific value Q of each of the 40 kinds of sample mixed gas was calculated by using the created calorific-value equations. The calculated calorific value Q was compared with the true calorific value Q. As shown in Fig. 22, an error was plus/minus 1.3% or less. Meanwhile, resistance of the temperature detector was not changed and, at the same time, resistance of the heating element was decreased by 0.03%, 0.07%, and 0.10% on purpose. Then, the error was increased. It indicated that drift of the heating element due to aging degradation or the like affects calculation of a calorific value.

As described above, current is supplied to the temperature detector such that the temperature detector does not produce heat by itself. Because of this, aging degradation of the temperature detector is less than aging degradation of the heating element. In the case of calculating a calorific value based on the calorific-value equation including the electric signal $S_I$ output from the temperature detector, it is possible to accurately calculate the calorific value before the heating element is aged and degraded. However, after the heating element is aged and degraded, an error of a calculated calorific value is generated. The error of a calculated calorific value is due to difference between aging degradation of the heating element and aging degradation of the temperature detector.

(Example 2)

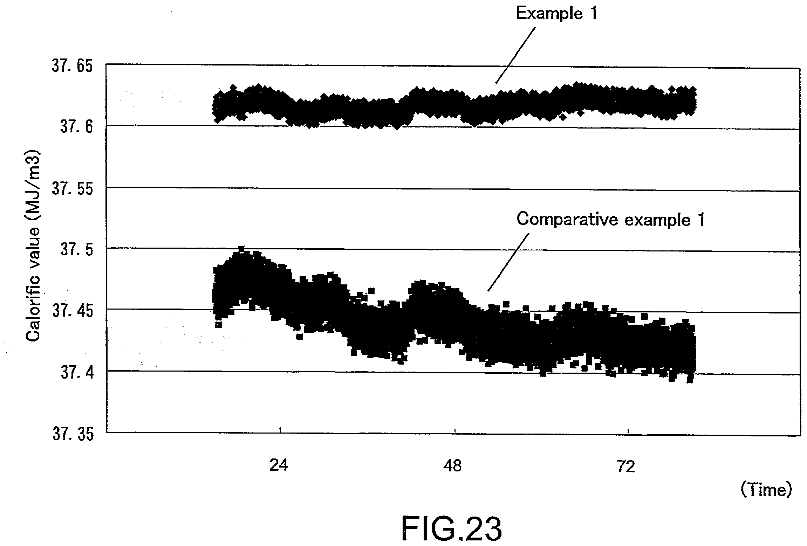

The calorific value of methane gas was temporally calculated based on the calorific-value equation created in Example 1. The calorific-value equation created in Example 1 did not include the electric signal $S_I$ output from the temperature detector as an independent variable. Further, the calorific value of methane gas was temporally calculated based on the calorific-value equation created in the comparative example. The calorific-value equation created in the comparative example included the electric signal $S_I$ output from the temperature detector as an independent variable. As a result, as shown in Fig. 23, in the case of using the calorific-value equation created in Example 1, the calculated calorific value of methane gas was approximately constant. However, in the case of using the calorific-value equation created in the comparative example, the calculated calorific value of methane gas was decreased as time passed.

(Example 3)

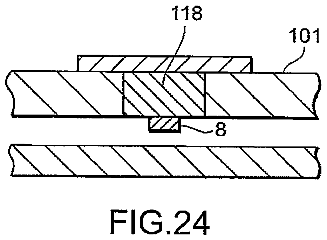

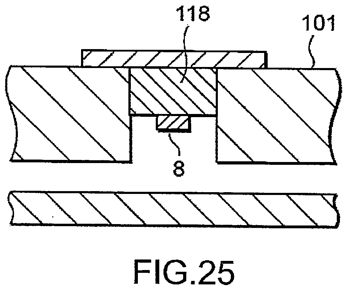

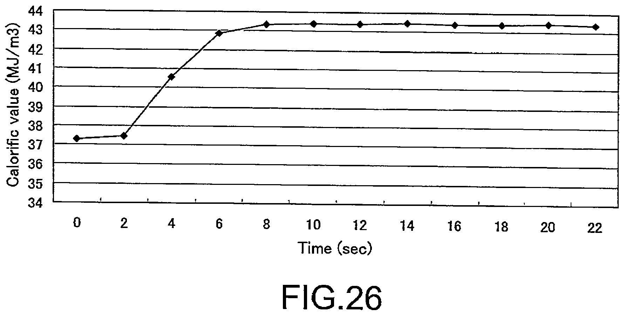

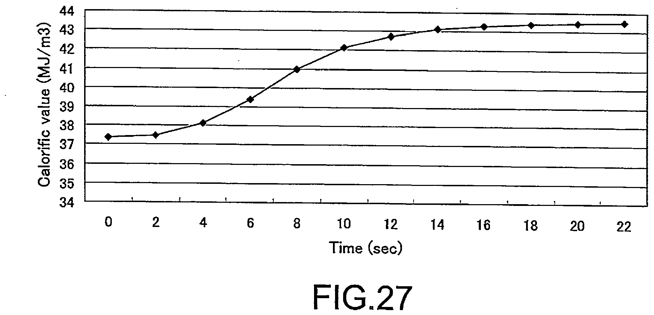

A calorific-value measuring system of Example 3 was prepared. As shown in Fig. 24, in Example 3, the microchip 8 was arranged such that the microchip 8 projected in the pipe 101. Further, a calorific-value measuring system of a comparative example was prepared. As shown in Fig. 25, in the comparative example, a concave was provided in the side wall of the pipe 101, and the microchip 8 was arranged in the concave of the side wall of the pipe 101. Next, the calorific value of measuring-target mixed gas was calculated by using the calorific-value measuring system of Example 3 under a particular condition. The calorific value of measuring-target mixed gas was calculated by using the calorific-value measuring system of the comparative example under the same condition. Then, as shown in Fig. 26, the calorific-value measuring system of Example 3 completed calculation of the calorific value in about 8 seconds. However, as shown in Fig. 27, the calorific-value measuring system of the comparative example completed calculation of the calorific value in about 16 seconds. It thus indicated that, by arranging the microchip 8 such that the microchip 8 projected in the pipe 101, responsivity of the microchip 8 was increased.

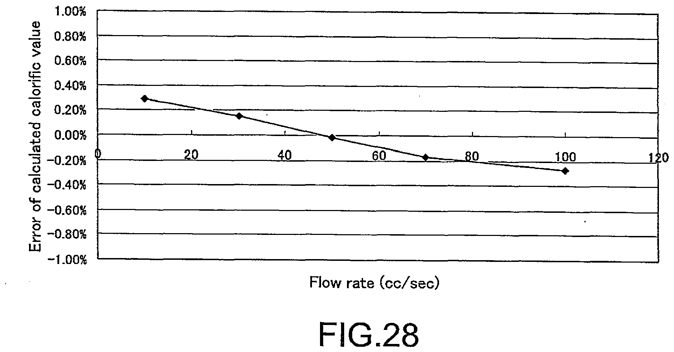

Further, the flow rate was variously changed, and the calorific value of measuring-target mixed gas was calculated by using the calorific-value measuring system of Example 3. As a result, as shown in Fig. 28, even if the flow rate was changed, a calculation error of the calorific value was 0.3% or less. It thus indicated that the calorific-value measuring system of Example 3 was capable of accurately calculating the calorific value under an environment in which it is not desirable to change the flow rate of gas. Examples of such an environment include a path for supplying gas to a gas turbine.

**Claims**

1.  A calorific-value-equation creating system, comprising:

    a pipe (101) in which each of a plurality of kinds of mixed gas can flow;
    a flow controller (32A, 32B, 32C, 32D) configured to control a flow rate of each of the plurality of kinds of mixed gas flowing in the pipe (101);
    a heating element (61) configured to produce heat at a plurality of heating temperatures ($T_H$), the heating element (61) being arranged in the pipe (101);
    a measuring section (301) configured to measure values of electric signals ($S_H$) output from the heating element, the heating element (61) being exposed to each of the plurality of kinds of mixed gas flowing in the pipe (101), the heating element (61) producing heat at the plurality of heating temperatures ($T_H$), respectively; and
    an equation creating section (302) configured to create a calorific-value equation (38) including independent variables and a dependent variable based on known calorific values of the plurality of kinds of mixed gas and only based on measured values of electric signals ($S_{H1}(T_{H1})$, $S_{H2}(T_{H2})$, ..., ($S_{Hn-1}(T_{Hn-1})$)) output from the heating element (61) at the plurality of heating temperatures ($T_H$), but not based on electric signals output from a temperature detector (62; 63) depending on a temperature of gas flowing in the pipe, the independent variables being electric signals ($S_H$) output from the heating element (61) at the plurality of heating temperatures ($T_H$), respectively, the dependent variable being the calorific value (Q).

2.  The calorific-value-equation creating system according to claim 1, wherein
    the heating element (61) is arranged such that the heating element projects in the pipe (101),
    wherein the heating element (61) is preferably arranged such that the heating element projects in the pipe (101) obliquely and such that the heating element (61) faces each of the plurality of kinds of mixed gas flowing in the pipe (101).

3.  The calorific-value-equation creating system according to claim 1 or 2, wherein
    the number of the plurality of heating temperatures ($T_{H1}$, $T_{H2}$, .., $T_{Hn-1}$) of the heating element (61) is at least a value obtained by subtracting 1 from the number of gas components in each of the plurality of kinds of mixed gas.

4.  The calorific-value-equation creating system according to any one of claims 1 to 3, wherein
    the equation creating section (302) is configured to create the calorific-value equation (38) by means of support vector regression.

5.  A calorific-value-equation creating method, comprising:

    causing each of a plurality of kinds of mixed gas to flow in a pipe (101);
    controlling a flow rate of each of the plurality of kinds of mixed gas flowing in the pipe (101);
    causing a heating element (61) to produce heat at a plurality of heating temperatures ($T_{H1}$, $T_{H2}$, .., $T_{Hn-1}$), the heating element (61) being exposed to each of the plurality of kinds of mixed gas flowing in the pipe (101);
    measuring values of electric signals ($S_{H1}(T_{H1})$, $S_{H2}(T_{H2})$, ..., ($S_{Hn-1}(T_{Hn-1})$)) output from the heating element (61) at the plurality of heating temperatures ($T_{H1}$, $T_{H2}$, .., $T_{Hn-1}$), respectively; and

creating a calorific-value equation (38) including independent variables and a dependent variable based on known calorific values of the plurality of kinds of mixed gas and only based on the measured values of electric signals ($S_{H1}(T_{H1})$, $S_{H2}(T_{H2})$, ..., $(S_{Hn-1}(T_{Hn-1})$) output from the heating element (61) at the plurality of heating temperatures ($T_{H1}$, $T_{H2}$, .., $T_{Hn-1}$), but not based on electric signals output from a temperature detector (62; 63) depending on a temperature of gas flowing in the pipe, the independent variables being electric signals ($S_H$) output from the heating element (61) at the plurality of heating temperatures ($T_H$), respectively, the dependent variable being the calorific value (Q).

6. The calorific-value-equation creating method according to claim 5, wherein
the heating element (61) is arranged such that the heating element projects in the pipe (101),
wherein the heating element (61) is preferably arranged such that the heating element projects in the pipe (101) obliquely and such that the heating element (61) faces each of the plurality of kinds of mixed gas flowing in the pipe (101).

7. The calorific-value-equation creating method according to claim 5 or 6, wherein
the number of the plurality of heating temperatures ($T_{H1}$, $T_{H2}$, .., $T_{Hn-1}$) is at least a value obtained by subtracting 1 from the number of gas components in each of the plurality of kinds of mixed gas.

8. The calorific-value-equation creating method according to any one of claims 5 to 7, wherein
support vector regression is used in the step of creating the calorific-value equation (38).

9. A calorific-value measuring system (20:), comprising:

a pipe (101) in which measuring-target mixed gas can flow;
a flow controller configured to control a flow rate of the measuring-target mixed gas flowing in the pipe (101);
a heating element (61) configured to produce heat at a plurality of heating temperatures ($T_{H1}$, $T_{H2}$, $T_{H3}$), the heating element (61) being arranged in the pipe (101);
a measuring section (301) configured to measure values of electric signals ($S_{H1}(T_{H1})$, $S_{H2}(T_{H2})$, $S_{H3}(T_{H3})$ output from the heating element (61), the heating element being exposed to the measuring-target mixed gas flowing in the pipe (101), the heating element producing heat at the plurality of heating temperatures ($T_{H1}$, $T_{H2}$, $T_{H3}$), respectively;
an equation storage device (402) configured to store a calorific-value equation (38) including independent variables and a dependent variable, the independent variables being only electric signals ($S_H$) output from the heating element at the plurality of heating temperatures ($T_H$), respectively, the dependent variable being the calorific value (Q); and
a calorific-value calculating section (305) configured to substitute only the measured values of the electric signals ($S_{H1}(T_{H1})$, $S_{H2}(T_{H2})$, $S_{H3}(T_{H3})$ output from the heating element (61) in the independent variables ($S_H$) of the calorific-value equation (38), respectively, but not values of electric signals output from a temperature detector (62; 63) depending on a temperature of gas flowing in the pipe, to calculate a calorific value (Q) of the measuring-target mixed gas.

10. The calorific-value measuring system according to claim 9, wherein
the heating element (61) is arranged such that the heating element projects in the pipe (101),
wherein the heating element (61) is preferably arranged such that the heating element projects in the pipe (101) obliquely and such that the heating element (61) faces the measuring-target mixed gas flowing in the pipe (101).

11. The calorific-value measuring system according to claim 9 or 10, wherein
the number of the plurality of heating temperatures ($T_{H1}$, $T_{H2}$, $T_{H3}$) is at least a value obtained by subtracting 1 from the number of a plurality of kinds of gas components in the measuring-target mixed gas.

12. The calorific-value measuring system according to any one of claims 9 to 11, comprising a calorific-value equation system according to any of claims 1 to 5 for creating the calorific-value equation (402) to be stored in the equation storage device (402).

13. A calorific-value measuring method, comprising:

causing measuring-target mixed gas to flow in a pipe (101);

controlling a flow rate of the measuring-target mixed gas flowing in the pipe (101);
causing a heating element (61) to produce heat at a plurality of heating temperatures ($T_{H1}$, $T_{H2}$, $T_{H3}$), the heating element (61) being exposed to the measuring-target mixed gas flowing in the pipe (101);
measuring values of electric signals ($S_{H1}(T_{H1})$, $S_{H2}(T_{H2})$, $S_{H3}(T_{H3})$) output from the heating element (61) at the plurality of heating temperatures ($T_{H1}$, $T_{H2}$, $T_{H3}$), respectively;
providing a calorific-value equation (38) including independent variables and a dependent variable, the independent variables only being electric signals ($S_H$) output from the heating element (61) at the plurality of heating temperatures ($T_H$), respectively, the dependent variable being a calorific value (Q); and
substituting only the measured values of the electric signals ($S_{H1}(T_{H1})$, $S_{H2}(T_{H2})$, $S_{H3}(T_{H3})$) output from the heating element (61) in the independent variables ($S_H$) of the calorific-value equation) (38), respectively, but not values of electric signals output from a temperature detector (62; 63) depending on a temperature of gas flowing in the pipe, to calculate a calorific value (Q) of the measuring-target mixed gas.

**14.** The calorific-value measuring method according to claim 13, wherein
the heating element (61) is arranged such that the heating element projects in the pipe (101),
wherein the heating element is preferably arranged such that the heating element (61) projects in the pipe obliquely and such that the heating element (61) faces the measuring-target mixed gas flowing in the pipe (101).

**15.** The calorific-value measuring method according to claim 12 or 13, wherein
the number of the plurality of heating temperatures ($T_{H1}$, $T_{H2}$, $T_{H3}$) is at least a value obtained by subtracting 1 from the number of a plurality of kinds of gas components in the measuring-target mixed gas.

**16.** The calorific-value measuring method according to any one of claim 13 to 15, wherein
the calorific-value equation (38) is created according to the method of any of claims 5 to 8.


**Patentansprüche**

**1.** Brennwertgleichungs-Erzeugungssystem, umfassend:

ein Rohr (101), in welchem jede von mehreren Arten gemischten Gases strömen kann;
eine Strömungssteuerung (32A , 32B, 32C, 32D), konfiguriert zum Steuern eines Strömungsdurchsatzes jeder der mehreren Arten von gemischtem Gas, das in dem Rohr (101) strömt;
ein Heizelement (61), konfiguriert zum Erzeugen von Wärme bei mehreren Heiztemperaturen ($T_H$), wobei das Heizelement (61) in dem Rohr (101) angeordnet ist;
einen Messabschnitt (301), konfiguriert zum Messen von Werten elektrischer Signale ($S_H$), die von dem Heizelement (61) ausgegeben werden, welches jeder der mehreren Arten gemischten Gases, das in dem Rohr (101) strömt, ausgesetzt ist, wobei das Heizelement (61) Wärme an den mehreren Heiztemperaturen ($T_H$) erzeugt; und
einen Gleichungserzeugungsabschnitt (302), konfiguriert zum Erzeugen einer Brennwertgleichung (38), die unabhängige Variablen und eine abhängige Variable basierend auf bekannten Brennwerten der mehreren Arten von gemischtem Gas enthält und lediglich basierend auf Messwerten elektrischer Signale ($S_{H1}(T_{H1})$, $S_{H2}(T_{H2})$, ..., ($S_{Hn-1}(T_{Hn-1})$)), die von dem Heizelement (61) bei den mehreren Heiztemperaturen ($T_H$) ausgegeben werden, jedoch nicht basierend auf elektrischen Signalen, die von einem Temperaturdetektor (62; 63) abhängig von einer Temperatur in dem Rohr strömenden Gases ausgegeben werden,
wobei die unabhängigen Variablen elektrische Signale ($S_H$) sind, die von dem Heizelement (61) bei den mehreren Heiztemperaturen ($T_H$) ausgegeben werden, und die abhängige Variable der Brennwert (Q) ist.

**2.** System nach Anspruch 1, bei dem
das Heizelement (61) derart angeordnet ist, dass das Heizelement in das Rohr (101) vorsteht,
wobei das Heizelement (61) vorzugsweise derart angeordnet ist, dass das Heizelement schräg und derart in das Rohr vorsteht, dass das Heizelement (61) jeder der mehreren Arten von gemischtem Gas, das in dem Rohr (101) strömt, gegenübersteht.

**3.** System nach Anspruch 1 oder 2, bei dem
die Anzahl der mehreren Heiztemperaturen ($T_{H1}$, $T_{H2}$, ..., $T_{Hn-1}$) des Heizelements (61) mindestens ein Wert ist, den man erhält durch Subtrahieren von 1 von der Anzahl von Gaskomponenten in jeder der mehreren Arten von gemischtem Gas.

**4.** System nach einem der Ansprüche 1 bis 3, bei dem
der Gleichungserzeugungsabschnitt (302) konfiguriert ist zum Erzeugen der Brennwertgleichung (38) mittels Support-Vektor-Regression.

**5.** Brennwertgleichungs-Erzeugungsverfahren, umfassend:

Veranlassen jeder von mehreren Arten gemischten Gases, in einem Rohr (101) zu strömen;
Steuern eines Strömungsdurchsatzes jeder der mehreren Arten von gemischtem Gas, das in dem Rohr (101) strömt;
Veranlassen eines Heizelements (61) zum Erzeugen von Wärme bei mehreren Heiztemperaturen ($T_{H1}$, $T_{H2}$, ... $T_{Hn-1}$), wobei das Heizelement (61) jeder der mehreren Arten von gemischtem Gas, das in dem Rohr (101) strömt, ausgesetzt ist;
Messen von Werten elektrischer Signale ($S_{H1}(T_{H1})$, $S_{H2}(T_{H2})$, ... ($S_{Hn-1}(T_{Hn-1})$)), die von dem Heizelement (61) bei den mehreren Heiztemperaturen ($T_{H1}$, $T_{H2}$, ... $T_{Hn-1}$) abgegeben werden; und
Erzeugen einer Brennwertgleichung (38), die unabhängige Variablen und eine abhängige Variable enthält, basierend auf bekannten Brennwerten mehrerer Arten von gemischtem Gas und nur basierend auf Messwerten elektrischer Signale ($S_{H1}(T_{H1})$, $S_{H2}(T_{H2})$, ..., ($S_{Hn-1}(T_{Hn-1})$)), die von dem Heizelement (61) bei den mehreren Heiztemperaturen ($T_H$) ausgegeben werden, jedoch nicht basierend auf elektrischen Signalen, die von einem Temperaturdetektor (62; 63) abhängig von einer Temperatur in dem Rohr strömenden Gases ausgegeben werden,
wobei die unabhängigen Variablen elektrische Signale ($S_H$) sind, die von dem Heizelement (61) bei den mehreren Heiztemperaturen ($T_H$) ausgegeben werden, und die abhängige Variable der Brennwert (Q) ist.

**6.** Verfahren nach Anspruch 5, bei dem
das Heizelement (61) derart angeordnet ist, dass das Heizelement in das Rohr (101) vorsteht,
wobei das Heizelement (61) vorzugsweise derart angeordnet ist, dass das Heizelement schräg und derart in das Rohr vorsteht, dass das Heizelement (61) jeder der mehreren Arten von gemischtem Gas, das in dem Rohr (101) strömt, gegenübersteht.

**7.** Verfahren nach Anspruch 5 oder 6, bei dem
die Anzahl der mehreren Heiztemperaturen ($T_{H1}$, $T_{H2}$, ..., $T_{Hn-1}$) des Heizelements (61) mindestens ein Wert ist, den man erhält durch Subtrahieren von 1 von der Anzahl von Gaskomponenten in jeder der mehreren Arten von gemischtem Gas.

**8.** Verfahren nach einem der Ansprüche 5 bis 7, bei dem
Support-Vektor-Regression in dem Schritt des Erzeugens der Brennwertgleichung (38) verwendet wird.

**9.** Brennwertmesssystem (20), umfassend:

ein Rohr (101), in welchem gemischtes Gas als Messobjekt strömen kann;
eine Strömungssteuerung, konfiguriert zum Steuern eines Strömungsdurchsatzes des das Messobjekt bildenden gemischtes Gases, das in dem Rohr (101) strömt;
ein Heizelement (61), konfiguriert zum Erzeugen von Wärme bei mehreren Heiztemperaturen ($T_{H1}$, $T_{H2}$, $T_{H3}$), angeordnet innerhalb des Rohrs (101);
einen Messabschnitt (301), konfiguriert zum Messen von Werten von elektrischen Signalen ($S_{H1}(T_{H1})$, $S_{H2}(T_{H2})$, $S_{H3}(T_{H3})$), die von dem Heizelement (61) ausgegeben werden, wobei das Heizelement dem das Messobjekt bildenden gemischten Gas, das in dem Rohr (101) strömt, ausgesetzt ist und das Heizelement Wärme bei den mehreren Heiztemperaturen ($T_{H1}$, $T_{H2}$, $T_{H3}$) erzeugt;
eine Gleichungsspeichereinrichtung (401), konfiguriert zum Speichern einer Brennwertgleichung (38), die unabhängige Variablen und eine abhängige Variable enthält, wobei die unabhängigen Variablen nur elektrische Signale ($S_H$) sind, die von dem Heizelement bei den mehreren Heiztemperaturen ($T_H$) ausgegeben werden, während die abhängige Variable der Brennwert (Q) ist; und
einen Brennwert-Berechnungsabschnitt (305), konfiguriert zum Ersetzen lediglich der Messwerte der elektrischen Signale ($S_{H1}(T_{H1})$, $S_{H2}(T_{H2})$, $S_{H3}(T_{H3})$), die von dem Heizelement (61) ausgegeben werden, in den unabhängigen Variablen ($S_H$) der Brennwertgleichung (38), nicht jedoch Werte von elektrischen Signalen, die von einem Temperaturdetektor (62; 63) abhängig von einer Temperatur von in dem Rohr strömendem Gas ausgegeben werden, um einen Brennwert (Q) des das Messobjekt bildenden gemischten Gases zu berechnen.

**10.** Messsystem nach Anspruch 9, bei dem
das Heizelement (61) derart angeordnet ist, dass das Heizelement in das Rohr (101) vorsteht,
wobei das Heizelement (61) vorzugsweise derart angeordnet ist, dass das Heizelement schräg und derart in das Rohr vorsteht, dass das Heizelement (61) jeder der mehreren Arten von gemischtem Gas, das in dem Rohr (101) strömt, gegenübersteht.

**11.** Messsystem nach Anspruch 9 oder 10, bei dem
die Anzahl der mehreren Heiztemperaturen ($T_{H1}$, $T_{H2}$, ..., $T_{Hn-1}$) des Heizelements (61) mindestens ein Wert ist, den man erhält durch Subtrahieren von 1 von der Anzahl von Gaskomponenten in jeder der mehreren Arten von gemischtem Gas.

**12.** Messsystem nach einem der Ansprüche 9 bis 11, enthaltend ein Brennwertgleichungssystem nach einem der Ansprüche 1 bis 5 zum Erzeugen der Brennwertleistung (402) zur Speicherung in der Gleichungsspeichereinrichtung (401).

**13.** Brennwert-Messverfahren, umfassend:

Veranlassen eines ein Messobjekt bildenden gemischten Gases, in einem Rohr (101) zu strömen;
Steuern eines Strömungsdurchsatzes des das Messobjekt bildenden gemischten Gases, das in dem Rohr (101) strömt;
Veranlassen eines Heizelements (61), Wärme mit mehreren Heiztemperaturen ($T_{H1}$, $T_{H2}$, $T_{H3}$) zu erzeugen, wobei das Heizelement (61) den das Messobjekt bildenden gemischten Gas ausgesetzt ist, das in dem Rohr (101) strömt;
Messen von Werten elektrischer Signale ($S_{H1}(T_{H1})$, $S_{H2}(T_{H2})$, $S_{H3}(T_{H3})$), die von dem Heizelement (61) bei den mehreren Heiztemperaturen ($T_{H1}$, $T_{H2}$, $T_{H3}$) ausgegeben werden;
Bereitstellen einer Brennwertgleichung (38), die unabhängige Variablen und eine abhängige Variable enthält, wobei die unabhängigen Variablen lediglich elektrische Signale ($S_H$) sind, die von dem Heizelement (61) bei den jeweiligen mehreren Heiztemperaturen ($T_H$) ausgegeben werden, während die abhängige Variable ein Brennwert ($Q$) ist; und
Einsetzen lediglich der Messwerte der elektrischen Signale ($S_{H1}(T_{H1}$, $S_{H2}(T_{H2})$, $S_{H3}(T_{H3})$, die von dem Heizelement (61) ausgegeben werden, in die unabhängigen Variablen ($S_H$) der Brennwertgleichung (38), nicht jedoch Werte von elektrischen Signalen, die von einem Temperaturdetektor (62; 63) abhängig von einer Temperatur von in dem Rohr strömendem Gas ausgegeben werden, um einen Brennwert ($Q$) des das Messobjekt bildenden gemischten Gases zu berechnen.

**14.** Verfahren nach Anspruch 13, bei dem
das Heizelement (61) derart angeordnet ist, dass das Heizelement in das Rohr (101) vorsteht,
wobei das Heizelement (61) vorzugsweise derart angeordnet ist, dass das Heizelement schräg und derart in das Rohr vorsteht, dass das Heizelement (61) jeder der mehreren Arten von gemischtem Gas, das in dem Rohr (101) strömt, gegenübersteht.

**15.** Verfahren nach Anspruch 12 oder 13, bei dem
die Anzahl der mehreren Heiztemperaturen ($T_{H1}$, $T_{H2}$, ..., $T_{Hn-1}$) des Heizelements (61) mindestens ein Wert ist, den man erhält durch Subtrahieren von 1 von der Anzahl von Gaskomponenten in jeder der mehreren Arten von gemischtem Gas.

**16.** Verfahren nach einem der Ansprüche 13 bis 15, bei dem
die Brennwertgleichung (38) gemäß dem Verfahren nach einem der Ansprüche 5 bis 8 erzeugt wird.

**Revendications**

**1.** Système de création d'équation de valeur calorifique, comprenant :

une conduite (101), dans laquelle chacun d'une pluralité de types de gaz mixte peut circuler ;
un contrôleur de flux (32A, 32B, 32C, 32D) configuré pour contrôler un débit de chacun de la pluralité de types de gaz mixte circulant dans la conduite (101) ;
un élément chauffant (61) configuré pour produire de la chaleur à une pluralité de températures de chauffage

($T_H$), l'élément chauffant (61) étant agencé dans la conduite (101) ;
une section de mesure (301) configurée pour mesurer des valeurs de signaux électriques ($S_H$) produits à partir de l'élément chauffant, l'élément chauffant (61) étant exposé à chacun de la pluralité de types de gaz mixte circulant dans la conduite (101), l'élément chauffant (61) produisant de la chaleur respectivement à la pluralité de températures de chauffage ($T_H$), et
une section de création d'équation (302) configurée pour créer une équation de valeur calorifique (38) incluant des variables indépendantes et une variable dépendante sur la base de valeurs calorifiques connues de la pluralité de types de gaz mixte et uniquement sur la base de valeurs mesurées de signaux électriques ($S_{H1}(T_{H1})$, $S_{H2}(T_{H2})$, ..., ($S_{Hn-1}(T_{Hn-1})$)) produits à partir de l'élément chauffant (61) à la pluralité de températures de chauffage ($T_H$), et non pas sur la base de signaux électriques produits à partir d'un détecteur de température (62; 63) dépendant d'une température de gaz circulant dans la conduite, les variables indépendantes étant des signaux électriques ($S_H$) produits à partir de l'élément chauffant (61) respectivement à la pluralité de températures de chauffage ($T_H$), la variable dépendante étant la valeur calorifique ($Q$).

2. Système de création d'équation de valeur calorifique selon la revendication 1, dans lequel
l'élément chauffant (61) est agencé de telle sorte que l'élément chauffant fait saillie dans la conduite (101), et dans lequel l'élément chauffant (61) est de préférence agencé de telle sorte que l'élément chauffant fait saillie dans la conduite (101) de manière oblique, et de telle sorte que l'élément chauffant (61) fait face à chacun de la pluralité de types de gaz mixte circulant dans la conduite (101).

3. Système de création d'équation de valeur calorifique selon la revendication 1 ou 2, dans lequel
le nombre de la pluralité de températures de chauffage ($T_{H1}$, $T_{H2}$, .., $T_{Hn-1}$) de l'élément chauffant (61) est au moins une valeur obtenue en soustrayant 1 du nombre de composants de gaz dans chacun de la pluralité de types de gaz mixte.

4. Système de création d'équation de valeur calorifique selon l'une quelconque des revendications 1 à 3, dans lequel :
la section de création d'équation (302) est configurée pour créer l'équation de valeur calorifique (38) au moyen d'une régression de vecteur de support.

5. Procédé de création d'équation de valeur calorifique, comprenant les étapes consistant à :

faire en sorte que chacun d'une pluralité de types de gaz mixte circule dans une conduite (101) ;
contrôler un débit de chacun de la pluralité de types de gaz mixte circulant dans la conduite (101) ;
faire en sorte qu'un élément chauffant (61) produise de la chaleur à une pluralité de températures de chauffage ($T_{H1}$, $T_{H2}$, .., $T_{Hn-1}$), l'élément chauffant (61) étant exposé à chacun de la pluralité de types de gaz mixte circulant dans la conduite (101) ;
mesurer des valeurs de signaux électriques ($S_{H1}(T_{H1})$, $S_{H2}(T_{H2})$, ..., ($S_{Hn-1}(T_{Hn-1})$)) produits à partir de l'élément chauffant (61) respectivement à la pluralité de températures de chauffage (($T_{H1}$, $T_{H2}$, .., $T_{Hn-1}$), et
créer une équation de valeur calorifique (38) incluant des variables indépendantes et une variable dépendante sur la base de valeurs calorifiques connues de la pluralité de types de gaz mixte et uniquement sur la base des valeurs mesurées de signaux électriques ($S_{H1}(T_{H1})$, $S_{H2}(T_{H2})$, ..., ($S_{Hn-1}(T_{Hn-1})$)) produits à partir de l'élément chauffant (61) respectivement à la pluralité de températures de chauffage (($T_{H1}$, $T_{H2}$, .., $T_{Hn-1}$), et non pas sur la base de signaux électriques produits à partir d'un détecteur de température (62; 63) dépendant d'une température de gaz circulant dans la conduite, les variables indépendantes étant des signaux électriques ($S_H$) produits à partir de l'élément chauffant (61) respectivement à la pluralité de températures de chauffage ($T_H$), la variable dépendante étant la valeur calorifique ($Q$).

6. Procédé de création d'équation de valeur calorifique selon la revendication 5, dans lequel
l'élément chauffant (61) est agencé de telle sorte que l'élément chauffant fait saillie dans la conduite (101), et dans lequel l'élément chauffant (61) est de préférence agencé de telle sorte que l'élément chauffant fait saillie dans la conduite (101) de manière oblique, et de telle sorte que l'élément chauffant (61) fait face à chacun de la pluralité de types de gaz mixte circulant dans la conduite (101).

7. Procédé de création d'équation de valeur calorifique selon la revendication 5 ou 6, dans lequel
le nombre de la pluralité de températures de chauffage ($T_{H1}$, $T_{H2}$, .., $T_{Hn-1}$) est au moins une valeur obtenue en soustrayant 1 du nombre de composants de gaz dans chacun de la pluralité de types de gaz mixte.

8. Procédé de création d'équation de valeur calorifique selon l'une quelconque des revendications 5 à 7, dans lequel

la régression de vecteur de support est utilisée lors de l'étape pour créer l'équation de valeur calorifique (38).

9. Système de mesure de valeur calorifique (20), comprenant :

une conduite (101), dans laquelle un gaz mixte cible de mesure peut circuler ;

un contrôleur de flux configuré pour contrôler un débit du gaz mixte cible de mesure circulant dans la conduite (101) ;

un élément chauffant (61) configuré pour produire de la chaleur à une pluralité de températures de chauffage ($T_{H1}$, $T_{H2}$, $T_{H3}$), l'élément chauffant (61) étant agencé dans la conduite (101) ;

une section de mesure (301) configurée pour mesurer des valeurs de signaux électriques ($S_{H1}(T_{H1})$, $S_{H2}(T_{H2})$, $S_{H3}(T_{H3})$ produits à partir de l'élément chauffant (61), l'élément chauffant étant exposé au gaz mixte cible de mesure circulant dans la conduite (101), l'élément chauffant produisant de la chaleur respectivement à la pluralité de températures de chauffage ($T_{H1}$, $T_{H2}$, $T_{H3}$) ;

un dispositif de stockage d'équation (402) configuré pour stocker une équation de valeur calorifique (38) incluant des variables indépendantes et une variable dépendante, les variables indépendantes étant uniquement des signaux électriques ($S_H$) produits à partir de l'élément chauffant respectivement à la pluralité de températures de chauffage ($T_H$), la variable dépendante étant la valeur calorifique (Q), et

une section de calcul de valeur calorifique (305) configurée pour substituer uniquement les valeurs mesurées des signaux électriques ($S_{H1}(T_{H1})$, $S_{H2}(T_{H2})$, $S_{Hn3}(T_{H3})$ produits à partir de l'élément chauffant (61) respectivement dans les variables indépendantes ($S_H$) de l'équation de valeur calorifique (38), et non pas de valeurs de signaux électriques produits à partir d'un détecteur de température (62;63) dépendant d'une température de gaz circulant dans la conduite, afin de calculer une valeur calorifique (Q) du gaz mixte cible de mesure.

10. Système de mesure de valeur calorifique selon la revendication 9, dans lequel l'élément chauffant (61) est agencé de telle sorte que l'élément chauffant fait saillie dans la conduite (101), et dans lequel l'élément chauffant (61) est de préférence agencé de telle sorte que l'élément chauffant fait saillie dans la conduite (101) de manière oblique, et de telle sorte que l'élément chauffant (61) fait face au gaz mixte cible de mesure circulant dans la conduite (101).

11. Système de mesure de valeur calorifique selon la revendication 9 ou 10, dans lequel le nombre de la pluralité de températures de chauffage ($T_{H1}$, $T_{H2}$, $T_{H3}$) est au moins une valeur obtenue en soustrayant 1 du nombre d'une pluralité de types de composants de gaz dans le gaz mixte cible de mesure.

12. Système de mesure de valeur calorifique selon l'une quelconque des revendications 9 à 11, comprenant un système d'équation de valeur calorifique selon l'une quelconque des revendications 1 à 5 pour créer l'équation de valeur calorifique (402) à stocker dans le dispositif de stockage d'équation (402).

13. Procédé de mesure de valeur calorifique, comprenant les étapes consistant à :

faire en sorte que le gaz mixte cible de mesure circule dans une conduite (101) ;

contrôler un débit du gaz mixte cible de mesure circulant dans la conduite (101) ;

faire en sorte qu'un élément chauffant (61) produise de la chaleur à une pluralité de températures de chauffage ($T_{H1}$, $T_{H2}$, $T_{H3}$), l'élément chauffant (61) étant exposé au gaz mixte cible de mesure circulant dans la conduite (101) ;

mesurer des valeurs de signaux électriques ($S_{H1}(T_{H1})$, $S_{H2}(T_{H2})$, $S_{H3}(T_{H3})$) produits à partir de l'élément chauffant (61) respectivement à la pluralité de températures de chauffage (($T_{H1}$, $T_{H2}$, $T_{H3}$) ;

fournir une équation de valeur calorifique (38) incluant des variables indépendantes et une variable dépendante, les variables indépendantes étant uniquement des signaux électriques ($S_H$) produits à partir de l'élément de chauffage (61) respectivement à la pluralité de températures de chauffage ($T_H$), la variable dépendante étant une valeur calorifique (Q), et

remplacer uniquement les valeurs mesurées des signaux électriques ($S_{H1}(T_{H1})$, $S_{H2}(T_{H2})$, $S_{H3}(T_{H3})$ produits à partir de l'élément chauffant (61) respectivement dans les variables indépendantes ($S_H$) de l'équation de valeur calorifique (38), et non pas de valeurs de signaux électriques produits à partir d'un détecteur de température (62; 63) dépendant d'une température de gaz circulant dans la conduite, afin de calculer une valeur calorifique (Q) du gaz mixte cible de mesure.

14. Procédé de mesure de valeur calorifique selon la revendication 13, dans lequel l'élément chauffant (61) est agencé de telle sorte que l'élément chauffant fait saillie dans la conduite (101), et

dans lequel l'élément chauffant (61) est de préférence agencé de telle sorte que l'élément chauffant fait saillie dans la conduite de manière oblique, et de telle sorte que l'élément chauffant (61) fait face au gaz mixte cible de mesure circulant dans la conduite (101).

15. Procédé de mesure de valeur calorifique selon la revendication 12 ou 13, dans lequel le nombre de la pluralité de températures de chauffage ($T_{H1}$, $T_{H2}$, $T_{H3}$) est au moins une valeur obtenue en soustrayant 1 du nombre d'une pluralité de types de composants de gaz dans le gaz mixte cible de mesure.

16. Procédé de mesure de valeur calorifique selon l'une quelconque des revendications 13 à 15, dans lequel l'équation de valeur calorifique (38) est créée conformément au procédé selon l'une quelconque des revendications 5 à 8.

FIG.1

8 : Microchip

EP 2 645 089 B1

8:Microchip

FIG.2

8

8 : Microchip

IV

IV

62

64

65

61

63

60

18

# FIG.3

8:Microchip

FIG.4

FIG.5

FIG.6

FIG.7

EP 2 645 089 B1

FIG.8

FIG.9

EP 2 645 089 B1

FIG.10

FIG.11

FIG.12

FIG.13

EP 2 645 089 B1

FIG.14

FIG.15

FIG.16

FIG.17

FIG.18

EP 2 645 089 B1

```
                    ┌─────────────┐
                    │    START    │
                    └─────────────┘
                           │
         ┌────────────────►▼
         │     ┌──────────────────────────────┐
         │     │    Prepare sample mixed gas   │─── S100
         │     └──────────────────────────────┘
         │            │
         │   ┌───────►▼
         │   │ ┌──────────────────────────────┐
         │   │ │       Output electric signal  │─── S101
         │   │ └──────────────────────────────┘
         │   │        │
         │   │        ▼                        S102
         │   │ No  ◇──────────────────────◇
         │   └────◇  Is temperature changed? ◇
         │        ◇──────────────────────◇
         │               │ Yes
         │               ▼                     S103
         │ No  ◇──────────────────────────◇
         └────◇ Is sample mixed gas changed? ◇
              ◇──────────────────────────◇
                     │ Yes
                     ▼
           ┌──────────────────────┐
           │      Collect data     │─── S104
           └──────────────────────┘
                     │
                     ▼
           ┌──────────────────────────┐
           │  Multiple regression analysis │─── S105
           └──────────────────────────┘
                     │
                     ▼
           ┌──────────────────────┐
           │       Register        │─── S106
           └──────────────────────┘
                     │
                     ▼
              ┌─────────────┐
              │     END     │
              └─────────────┘
```

FIG.19

```
                        ┌─────────────────┐
                        │      START      │
                        └─────────────────┘
                                 │
                                 ▼
              ┌──────────────────────────────────────┐
              │   Prepare measuring-target mixed gas  │────  S200
              └──────────────────────────────────────┘
                                 │
        ┌────────────────────────┤
        │                        ▼
        │     ┌──────────────────────────────────────┐
        │     │        Output electric signal         │────  S201
        │     └──────────────────────────────────────┘
        │                        │
        │                        ▼        ╭─ S202
        │              ╱─────────────────────╲
   No   │         ────╱   Is temperature changed?  ╲────
   ─────┘             ╲─────────────────────╱
                               │
                               ▼ Yes
              ┌──────────────────────────────────────┐
              │           Retrieve equation           │────  S203
              └──────────────────────────────────────┘
                               │
                               ▼
              ┌──────────────────────────────────────┐
              │        Calculate calorific value      │────  S204
              └──────────────────────────────────────┘
                               │
                               ▼
                        ┌─────────────────┐
                        │       END       │
                        └─────────────────┘
```

# FIG.20

FIG.21

FIG.22

FIG.23

EP 2 645 089 B1

FIG.24

EP 2 645 089 B1

EP 2 645 089 B1

FIG.25

FIG.26

FIG.27

FIG.28

**EP 2 645 089 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004514138 A
- JP 2012233859 A
- EP 1947450 A1
- JP 2012202739 B
- JP H05141999 B